# EUROPEAN PATENT SPECIFICATION

(11) **EP 1 486 500 B1**
(45) Date of publication and mention of the grant of the patent: **16.09.2009**
(21) Application number: 03708575.0
(22) Date of filing: 13.03.2003
(51) Int. Cl.: C07D 498/06, A61K 31/5383, A61P 31/04

(54) **10-(3-CYCLOPROPYLAMINOMETHYL-1-PYRROLIDINYL)PYRIDOBENZOXAZINECARBOXYLIC ACID DERIVATIVE EFFECTIVE AGAINST RESISTANT BACTERIA**
10-(3-CYCLO PROPYL AMINO METHYL-1-PYRROLIDINYL) PYRIDOBENZOXAZIN CARBONSÄURE DERIVAT MIT WIRKUNG GEGEN RESISTENTE BAKTERIEN
DERIVE D'ACIDE 10-(3-CYCLOPROPYLAMINOMETHYL-1-PYRROLIDINYL)PYRIDOBENZOXAZINECARBOXYLIQUE EFFICACE CONTRE LES BACTERIES RESISTANTES

(30) Priority: 18.03.2002 JP 2002074783; 20.12.2002 JP 2002369205
(43) Date of publication of application: 15.12.2004
(73) Proprietor: Kyorin Pharmaceutical Co., Ltd., Tokyo 101-8311 (JP)
(72) Inventor: ASAHINA, Yoshikazu, Shimotsuga-gun, Tochigi 329-0101 (JP); TAKEI, Masaya, Shimotsuga-gun, Tochigi 329-0101 (JP)
(74) Representative: Vossius & Partner
(86) International application number: PCT/JP2003/002967
(87) International publication number: WO 2003/078439

(56) References cited:
- EP-A- 0 265 230
- EP-A1- 0 900 793
- JP-A- 10 287 669
- JP-A- 62 155 282
- KAWAKAMI KATSUHIRO ET AL.: 'Antimycobacterial activities of novel levofloxacin analogues' ANTIMICROBIAL AGENTS AND CHEMOTHERAPY vol. 44, no. 8, 2000, pages 2126 - 2129, XP002969586

## Description

The present invention relates to novel 10-(3-cyclopropylaminomethyl-1-pyrrolidinyl)pyridobenzoxazine carboxylic acid derivatives, salts and hydrates thereof that, in addition to being safe and exhibiting strong antibacterial activities, are effective against drug-resistant bacteria that are less susceptible to conventional antibacterial agents.

Reference should be made to the following articles: Japanese Patent Laid-Open Publication No. Sho 57-46986 (Patent Article 1); Japanese Patent Laid-Open Publication No. Sho 61-204188 (Patent Article 2); Japanese Patent Laid-Open Publication No. Sho 62-155282 (Patent Article 3) ; Kawakami et al, Antimicrobial Agents and Chemotherapy,, 2000, 44, 2126-2129.

Since the development of norfloxacin, considerable effort has been made worldwide to develop quinolone carboxylic acid-based antibacterial agents, Which are also known as new quinolones and have how become important cures for infectious diseases.

The recent emergence of drug-resistant bacteria, such as methicillin-Resistant Staphylococcus aureus (MRSA), Penicillin-Resistant Streptococcus pneumoniae (PRSP), and vancomycin-Resistant Enterococcus (VRE), most of which are gram-positive bacteria, has posed a serious threat to the treatment of patients. Traditional quinolone carboxylic acid-based antibacterial agents have relatively weak antibacterial activities against gram-positive bacteria and thus are not considered as effective cures for the drug-resistant bacteria. Furthermore, the increasing incidence of Quinolone-Resistant Staphylococcus aureus (QRSA) makes the use of these drugs even more difficult.

While pyridobenzoxazine carboxylic acid-based antibacterial agents similar to the ones claimed in the present invention are described in, for example, Patent Articles 1, 2, and 3, hone of these agents offer sufficient antibacterial activity against gram-positive bacteria, nor are they described to have antibacterial activity against drug-resistant bacteria such as those described above.

It is therefore an objective of the present invention to provide novel pyridobenzoxazine carboxylic acid-based compounds that, in addition to being safe and exhibiting strong antibacterial activities, are effective against drug-resistant bacteria that are less susceptible to conventional antibacterial agents.

In view of the above-described problems, the present inventors have devoted a significant amount of effort to seeking quinolone carboxylic acid derivatives that are effective against gram-positive bacteria, in particular, such drug-resistant bacteria as MRSA, PRSP, and VRE, which are less susceptible to traditional quinolone carboxylic acid-based antibacterial agents. The effort was rewarded by the discovery of the compounds of the present invention, which proved to be effective against gram-positive bacteria, in particular, such drug-resistant bacteria as MRSA, PRSP, and VRE, and exhibit higher antibacterial activity as compared not only with traditional quinolone carboxylic acid-based antibacterial agents, but also with various other antibacterial agents. The discovery ultimately led the present inventors to complete the present invention.

According to the present invention, there is provided a Compound as represented by the following general formula (I), or a salt or a hydrate thereof: 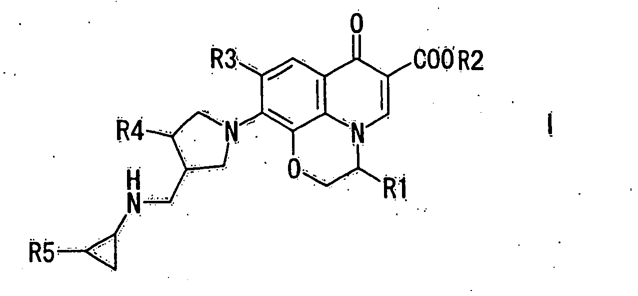 wherein R1 is a fluoromethyl group; R2 is a hydrogen atom, a lower alkyl group having 1 to 3 carbon atoms, or a pharmaceutically acceptable cation and an ester of a prodrug selected from a pivaloyloxymethyl group, an acetoxymethyl group, a phthalidinyl group, an indonyl group, a methoxymethyl group, and a 5-methyl-2-oxo-1,3-dioxolene-4-yl group ; R3 is a hydrogen atom or a halogen atom; R4 is a hydrogen atom, a lower alkyl group having 1 to 3 carbon atoms, a fluoromethyl group, a trifluoromethyl group or a fluorine atom; and R5 is a hydrogen atom or a fluorine atom.

Examples of the lower alkyl group in the general formula (I) include a methyl group, an ethyl group, a propyl group, an isopropyl group, and a cyclopropyl group. Examples of the pharmaceutically acceptable cation include sodium ion, potassium ion, magnesium ion, calcium ion, and ammonium ion. Examples of the halogen atom include fluorine, chlorine, bromine, and iodine.

An exemplary production process of the compound of the present invention will now be described.

The compound of the present invention may be produced by reacting a compound represented by the following general formula (II): 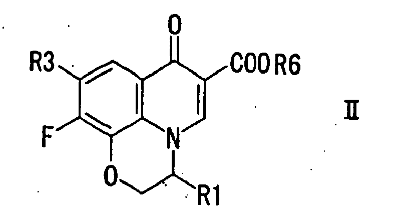 [wherein R1 and R3 are the same as in the general formula (I); and R6 is represented by the following general formula (III): 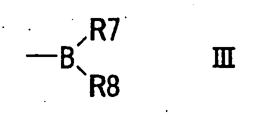 [wherein R6 and R7 are each independently a fluorine atom, or a lower alkylcarbonyloxy group]]
with a compound represented by the following general formula (IV), or an acid addition salt thereof: 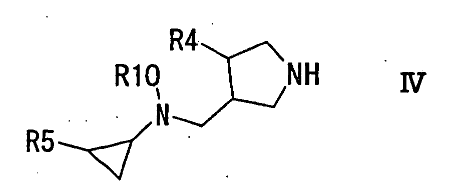 [wherein R4 and R5 are the same as in the general formula (I); and R10 is a hydrogen atom or a protective group of nitrogen atom such as t-butoxycarbonyl]
and then removing the boron chelate and, if necessary, the protective group of nitrogen atom.

The reaction of the compound of the general formula (II) with the compound of the general formula (IV) may be carried out in the absence or presence of a solvent, such as an alcohol, acetonitrile, dimethylsulfoxide, N,N-dimethylformamide, N,N-dimethylacetamide, N-methylpyrrolidone, tetrahydrofuran, dioxane, benzene, or toluene, and in the presence of an acid receptor. The acid receptor may be a carbonate or a hydrogen carbonate of an alkali metal or an alkaline earth metal, or a basic organic compound, such as triethylamine, diazabicyclo-7-undecene, or pyridine. The reaction is typically carried out at a temperature in the range of room temperature to 200°C and preferably in the range of 25°C to 150°C. The reaction takes from 30min to 48 hours and is typically complete within 30min to 15 hours.

If desired, the compound of the general formula (I) may be converted to its salt using an ordinary technique. Examples of such salts include salts formed with an inorganic acid, such as hydrochloric acid, sulfuric acid, and phosphoric acid, salts formed with an organic acid, such as methanesulfonic acid, lactic acid, oxalic acid, and acetic acid, and salts formed with sodium, potassium, magnesium, calcium, aluminum, cerium, chromium, cobalt, copper, iron, zinc, platinum, silver, or the like.

The compound of the present invention may be administered to humans or animals in a pharmaceutically known form through a pharmaceutically known route. For example, the compound may be prepared in the form of powders, tablets, capsules, ointments, injections, syrups, solutions, eye drops, and suppositories for oral or parenteral administration.

### EXAMPLES

Exemplary tests as well as production processes for the compound of the present invention will now be described in detail with reference to examples.

### Reference Example 1

### Bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶, O⁷] boron

To a mixture of boric acid (12.8g) and acetic anhydride (63.4g), zinc chloride (236mg) was added and the resulting mixture was stirred at room temperature for 0.5 hours. To this mixture, (3R)-9,10-difluoro-3-fluoromethyl-2,3-dihydro-7-oxo-7H-pyrido[1,2,3-d,e][1,4]benzoxazine-6-carboxylic acid ethyl ester (22.6g) was added and the mixture was stirred at 60°C for 2.5 hours. Subsequently, the reaction mixture was concentrated under reduced pressure and the resulting residue was dissolved in ethyl acetate (300mL). The solution was sequentially washed with a saturated aqueous solution of sodium hydrogen carbonate (2 x 200mL) and then with water (100mL), followed by drying over anhydrous sodium sulfate and concentration under reduced pressure. The resulting residue was purified on a silica gel column (dichloromethane: acetone = 7:1), and the eluted yellow amorphous product was crystallized in an acetone/diethyl ether mixture to give 24.5g of bis(acetato-O)[(3R)-9,10=difluoro-3-fluoromethyl-2,3-dihydro-7-oxo-7H-pyrido[1,2,3-d,e][1,4]benzoxazine-6-carboxylato-O⁶,O⁷] boron as a white powder.
NMR(CDCl₃): δ 1.85 (s, 3H), 2.05 (s, 3H), 4.62 (ddd, J = 2.9 Hz, 3.9 Hz, 12.2 Hz, 1H), 4.74 (ddd, J = 7.8 Hz, 10.3 Hz, 46.4 Hz, 1H), 4.90 (ddd, J = 4.9 Hz, 10. 3 Hz, 45.Hz, 1H), 4.92 (dd, J = 1.0 Hz, 12.7 Hz, 1H), 5.35-5.38 (m, 1H), 7.92 (dd, J = 7.3 Hz, 9.3 Hz, 1H), 9.22 (s, 1H).
Elementary analysis (%): Calcd. for C₁₇H₁₃BF₃NO₈·0.75H₂O : C 46.34, H 3.32, N 3.18; found: C 46.30, H 3.34, N 3.30

### Reference Example 2

### Synthesis of bis(acetato-O)[(3S)-9,10-difluoro-2,3-dihydro-3-methoxymethyl-7-oxo-7H-pyrido[1,2,3-d,e][1,4]benzoxazine-6-carboxylato-O⁶,O⁷]boron

### Step 1:

(3S)-9,10-Difluoro-2,3-dihydro-3-hydroxymethyl-7-oxo-7H-pyrido[1,2,3-d,e][1,4]benzoxazine-6-carboxylic acid ethyl ester (1.30g) was suspended in anhydrous dimethylformamide (40mL). Silver oxide (I) (4.63g) and methyl iodide (1.25mL) were then added to the suspension. The resulting mixture was stirred at room temperature for 21 hours. Subsequently, insoluble materials were removed from the reaction mixture by filtration and the filtrate was concentrated under reduced pressure. The resulting residue was purified on a silica gel column (dichloromethane: acetone= 5: 1) to give 740mg of (3S)-9,10-difluoro-2,3-dihydro-3-methoxymethyl-7-oxo-7H-pyrido[1,2,3-d,e][1,4]benzoxazine-6-carboxylic acid ethyl ester as a white powder.
MS(EI)m/z: 339 (M⁺)
Elementary analysis (%): Calcd. for C₁₆H₁₅F₂NO₅: C 56.64, H 4. 46, N 4.13; found: C 56.56, H 4.71, N 4.26.

### Step 2:

In a similar manner to Reference Example 1, (3S)-9,10 difluoro-2,3-dihydro-3-methoxymethyl-7-oxo-7H-pyrido[1,2,3-d,e][1,4]benzoxazine-6-carboxylic acid ethyl ester (679mg) was reacted to give 830mg of bis (acetato-O)[(3S)-9,10-difluoro-2,3-dihydro-3-methoxymethyl-7-oxo-7H-pyrido [1,2,3-d,e] [1,4]benzoxazine-6-carboxylato-O⁶, O⁷]boron as a colorless amorphous product.
¹H NMR(CDCl₃):δ 1.86 (s, 3H), 2.06 (s, 3H), 3.39 (s, 3H), 3.70 (dd, J =8.3 Hz, 10.3 Hz, 1H), 3.82 (dd, J = 5.4 Hz, 10.3 Hz, 1H), 4.56 (dd, J = 2.9 Hz, 12.4 Hz, 1H), 4.86 (dd, J = 1.0 Hz, 12.2 Hz, 1H), 5.10-5.13 (m, 1H), 7.89 (dd, J = 7.3 Hz, 9.3 Hz, 1H), 9.13 (s, 1H).
Elementary analysis (%): Calcd. for C₁₈H₁₆BF₂NO₉·1.5H₂O: C 46.38, H 4.11, N 3.00; found: C 46.18, H 3.74, N 311.

### Reference Example 3

### Synthesis of bis(acetato-O)[(3S)-3-acetoxymethyl-9,10-difluoro-2,3-dihydro-7-oxo-7H-pyrido[1,2,3-d,e][1,4]benzoxazine-6-carboxylato-O⁶,O⁷]boron

### Step 1:

(3S)-9,10-Difluoro-2,3-dihydro-3-hydroxymethyl-7-oxo-7H-pyrido[1,2,3-d,e][1,4]benzoxazine-6-carboxylic acid ethyl ester (976mg) was suspended in anhydrous dichloromethane (30mL). To the suspension, acetic anhydride (368mg) and 4-dimethylaminopyridine (5.0mg) were added and the resulting mixture was refluxed for 1.5 hours while heated. Subsequently, the mixture was allowed to cool and was washed with water. This was followed by drying over anhydrous sodium sulfate and concentration under reduced pressure. The resulting residue was suspended in ethanol and the suspension was filtrated to give 1.04g of (3R)-3-acetoxymethyl-9,10-difluoro-2,3-dihydro-7-oxo-7H-pyrido[1,2,3-d,e][1,4]benzoxazine-6-carboxylic acid ethyl ester as a white powder.
MS(EI)m/z: 367(M⁺).
Elementary analysis (%): Calcd. for C₁₇H₁₅F₂NO₆: C 55.59, H 4.12, N 3.81; found: C 56.25, H 4.15, N 3.93.

### Step 2:

In a similar manner to Reference Example 1, (3S)-3-acetoxymethyl-9,10-difluoro-2,3-dihydro-7-oxo-7H-pyrido[1,2,3-d,e][1,4]benzoxazine-6-carboxylic acid ethyl ester (918mg) was reacted to give 1.00g of bis(acetato-O)[(3S)-3-acetoxymethyl-9,10-difluoro-2,3-dihydro-7-oxo-7H-pyrido[1,2,3-d,e][1,4]benzoxazine-6-carboxylato-O⁶,O⁷]boron as a colorless amorphous product.
¹H NMR(CDCl₃): δ 1.83 (s, 3H), 2.03 (s, 3H), 2.08 (s, 3H), 4.44-4.54 (m, 2H), 4.63 (dd, J = 2.9 Hz, 12.2 Hz, 1H), 4.89 (dd, J = 1.0 Hz, 12.7 Hz, 1H), 5.27-5.30 (m, 1H), 7.88 (dd, J = 7.3 Hz, 9.3 Hz, 1H), 9.17 (s, 1H).
Elementary analysis (%): Calcd. for C₁₉H₁₆BF₂NO₁₀·1.75H₂O: C 45.76, H 3.94, N 2.81; found: C 45.94, H 3.82, N 2.95.

### Reference Example 4

### Synthesis of trans-3-cyclopropylaminomethyl-4-methylpyrrolidine

### Step 1:

trans-1-Benzyl-4-methyl-3-pyrrolidinecarboxylic acid (4.04g) was dissolved in dichloromethane (50mL). To this solution, 1,1'-carbonyl-bis-1H-imidazole (3.58g) was added and the mixture was stirred at room temperature for 1 hour. While the reaction mixture was cooled on an ice bath, a dichloromethane solution (15mL) of cyclopropylamine (1.53mL) was added dropwise and the mixture was stirred at room temperature for 3 hours. Subsequently, the reaction mixture was washed with water, was dried over anhydrous sodium sulfate, and was then concentrated under reduced pressure. The resulting residue was crystallized in a hexane/diisopropyl ether mixture and the formed crystals were filtrated. The collected crystals were then washed with a hexane/diisopropyl ether mixture and were dried under reduced pressure to obtain X 4.07g of trans-1-benzyl-N-cyclopropyl-4-methyl-3-pyrrolidinecarboxamide as white crystals.
Melting point: 81-83°C.
MS (EI) m/z: 258(M⁺).

### Step 2:

trans-1-Benzyl-N-cyclopropyl-4-methyl-3-pyrrolidinecarboxamide (3.80g) was suspended in anhydrous tetrahydrofuran (85mL). To this suspension, a 1mol/L tetrahydrofuran solution of borane-tetrahydrofuran complex (58.8mL) was added and the mixture was refluxed for 8 hours while heated. Subsequently, a 2mol/L aqueous solution of sodium hydroxide (35mL) was added to the reaction mixture and the mixture was refluxed for 4 hours while heated. After concentration under reduced pressure, the resultant residue was extracted with toluene (2 x 100mL) and the toluene extracts were combined. The combined extracts were washed with water, dried over anhydrous sodium sulfate, and then concentrated under reduced pressure. The resulting residue was dissolved in dichloromethane (50mL). To this solution, di-*tert*-butyl dicarbonate (3.53g) was added and the mixture was stirred at room temperature for 4 hours. Subsequently, the reaction mixture was concentrated under reduced pressure and the resulting residue was purified on a silica gel column (hexane: ethyl acetate = 4:1 shifted to 1:1) to obtain 3.07g of trans-1-benzyl-3-[[(N-tert-butoxycarbonyl-N-cyclopropyl)amino]methyl]-4-methylpyrrolidine as a colorless oil.
MS (FAB⁺)m/z: 345 (MH⁺).
HRMS (FAB⁺) : Calcd. for C₂₁H₃₃N₂O₂ (MH⁺): 345.2542; found: 345.2505.

### Step 3:

trans-1-Benzyl-3-[[(N-tert-butoxycarbonyl-N-cyclopropyl) amino]methyl]-4-methylpyrrolidine (3.00g) was dissolved in ethanol (50mL). To this solution, 7.5% palladium carbon (300mg) was added and the mixture was stirred at room temperature for 6 hours under a hydrogen pressure of 3.9 x 10⁵Pa. Subsequently, the catalyst was removed from the reaction mixture by filtration and the collected catalyst was washed with ethanol. The filtrate and the washing solution were combined and the resulting residue was dried under reduced pressure to obtain 2.12g of trans-3-[[(N-tert-butoxycarbonyl-N-cyclopropyl) amino]methyl]-4-methylpyrrolidine as a pale brown oil.
MS (FAB⁺) m/z: 255 (MH⁺). HRMS (FAB⁺): Calcd. for C₁₄H₂₇N₂O₂ (MH⁺): 255.2073; found: 255.2079.

### Step 4:

trans-3-[[(N-tert-Butoxycarbonyl-N-cyclopropyl)amino]methyl]-4-methylpyrrolidine (2.07g) was dissolved in dichloromethane (10mL). While this solution was cooled on an ice bath, trifluoroacetic acid (5mL) was added and the mixture was stirred at room temperature for 2 hours. After concentration under reduced pressure, the resulting residue was dissolved in tetrahydrofuran (6mL) and the solution was allowed to stand for 13 hours at room temperature. The separated crystals were collected by filtration, followed by washing with tetrahydrofuran and drying under reduced pressure, to give 2.47g of trans-3-cyclopropylaminomethyl-4-methylpyrrolidine trifluoroacetic acid salt. The salt product (2.37g) was dissolved in water (5mL), followed by addition of a 20% aqueous solution of sodium hydroxide to adjust the pH to 14. The solution was then extracted with diethyl ether (2 x 50mL) and the extracts were combined. The combined extracts were then dried over anhydrous sodium sulfate and concentrated under reduced pressure. The resulting residue was purified by distillation under reduced pressure to obtain 660mg of trans-3-cyclopropylaminomethyl-4-methylpyrrolidine.
¹H NMR(CDCl₃): δ 0.30-0.37 (m, 2H), 0.41-0.45(m, 2H), 1.04(d, J = 6.3 Hz, 3H), 1.66-1.76(m, 4H), 2.08-2.13(m, 1H), 2. 46 (dd, J = 7.3 Hz, 10.7 Hz, 1H), 2.57(dd, J = 8.3 Hz, 11.7 Hz, 1H), 2.63(dd, J = 6.3 Hz, 10.7 Hz, 1H), 2.80 (dd, J = 5.4 Hz, 11.7 Hz, 1H), 3.10 (dd, J = 6.8 Hz, 10.7Hz, 1H), 3.14 (dd, J = 7.3
Hz, 10.7 Hz, 1H).
Elementary analysis (%): Calcd. for C₉H₁₈N₂·2CF₃COOH: C 40.84, H 5.27, N 7.33; found: C 40.90, H 5.47, N 7.31.

### Reference Example 5

### Synthesis of (3R,4R)-3-cyclopropylaminomethyl-4-methylpyrrolidine

### Step 1:

(3R,4R)-1-Benzyl-4-methyl-3-pyrrolidinecarboxylic acid (6.27g) was suspended in dichloromethane (250mL). To this suspension, cyclopropylamine (1.76mL) and hydrochloric acid 1-ethyl-(3-dimethylaminopropyl)carbodiimide (12.2g) were sequentially added and the mixture was stirred at room temperature for 4 hours. Subsequently, the reaction mixture was washed with water, was dried over anhydrous sodium sulfate, and was then concentrated under reduced pressure. The resulting residue was purified on a silica gel column (ethyl acetate: methanol = 10:1) to give 3.32g of (3R,4R)-1-benzyl-N-cyclopropyl-4-methyl-3-pyrrolidinecarboxamide as white crystals.
MS (EI) m/z: 258 (M⁺).
Elementary analysis (%): Calcd. for C₁₆H₂₂N₂O.: C 74.38, H 8.58, N 10.84; found: C 74.46, H 8.67, N 10.72.

### Step 2:

In a similar manner to Step 2 in Reference Example 4, (3R, 4R)-1-benzyl-N-cyclopropyl-4-methyl-3-pyrrolidinecarboxamide (5.52g) was reacted to give 4.16g of (3R,4R)-1-benzyl-3-[[(N-tert-butoxycarbonyl-N-cyclopropyl)amino]methyl]-4-methylpyrrolidine as a pale brown oil.
MS (FAB⁺) m/z: 343 (MH⁺).
HRMS (FAB⁺): Calcd. for C₂₁H₃₃N₂O₂ (MH⁺): 345.2542; found 345.2585

### Step 3:

In a similar manner to Step 3 in Reference Example 4, (3R,4R)-1-benzyl-3-[[(N-tert-butoxycarbonyl-N-cyclopropyl)amino]methyl]-4-methylpyrrrolidine (4.00g) was reacted to give 2.88g of (3R, 4R)-3-[[(N-tert-butoxycarbonyl-N-cyclopropyl) amino] methyl]-4-methylpyrrolidine.
MS (FAS⁺) m/z: 255 (MH⁺).
HRMS (FAB⁺): Calcd. for C₁₄H₂₇N₂O₂(MH⁺): 255.2073; found: 255.2070.

### Step 4:

In a similar manner to Step 4 in Reference Example 4, (3R,4R)-3-[[(N-tert-butoxycarbonyl-N-cyclopropyl)amino]methyl]-4-methylpyrrolidine (2.78g) was reacted to give 730mg of (3R,4R)-3-cyclopropylaminomethyl-4-methylpyrrolidine.
Specific rotation: +74.6° (c = 0.648, methanol).
Elementary analysis (%): Calcd. for C₉H₁₈N₂ · 2CF₃COOH: C 40.84, H 5.27, N 7.33; found: C 40.73, H 5.26, N 7.36.

### Reference Example 6

### Synthesis of (3S,4S)-3-cyclopropylaminomethyl-4-methylpyrrolidine

### Step 1:

In a manner similar to Step 1 in Reference Example 5, (3S,4S)-1-benzyl-4-methyl-3-pyrrolidinecarboxylic acid (14.5g) was reacted to give 6.33g of (3S,4S)-1-benzyl-N-cyclopropyl-4-methyl-3-pyrrolidinecarboxamide as pale brown crystals.
MS (EI) m/z: 258 (M⁺).
Elementary analysis (%): Calcd. for C₁₆H₂₂N₂O: C 74.38, H 8.58, N 10.84; found: C 74.64, H 8.66, N 10.71.

### Step 2:

In a manner similar to Step 2 in Reference Example 4, (3S,4S)-1-benzyl-N-cyclopropyl-4-methyl-3-pyrrolidinecarboxamide (6.13g) was reacted to give 4.67g of (3S,4S)-1-benzyl-3-[[(N-tert-butoxycarbonyl-N-cyclopropyl)amino]methyl]-4-methylpyrrolidine as a pale brown oil.
MS (FAB⁺) m/z: 345 (MH⁺).
HRMS (FAB⁺): Calcd. for C₂₁H₃₃N₂O₂ (MH⁺): 345.2542; found: 345.2547.

### Step 3:

In a similar manner to Step 3 in Reference Example 4, (3S,4S)-1-benzyl-3-[[(N-tert-butoxycarbonyl-N-cyclopropyl)amino]methyl]-4-methylpyrrolidine (4.47g) was reacted to give 3.05g of (3S,4S)-3-[[(N-tert-butoxycarbonyl-N-cyclopropyl)amino]methyl]-4-methylpyrrolidine.
MS (FAB⁺) m/z: 255 (MH⁺)
HRMS (FAS⁺): Calcd. for C₁₄H₂₇N₂O₂ (MH⁺): 255.2073; found 255.2075.

### Step 4:

In a similar manner to Step 4 in Reference Example 4, (3S,4S)-3-[[(N-tert-butoxycarbonyl-N-cyclopropyl)amino]methyl]-4-methylpyrrolidine (2.85g) was reacted to give 1.21g of (3S,4S)-3-cyclopropylaminomethyl-4-methylpyrrolidine.
Specific rotation: -74.5° (c = 0.62, methanol).
Elementary analysis (%): Calcd. for C₉H₁₈N₂. 2CF₃COOH: C 40. 84, H 5.27, N 7.33; found: C 40.80, H 5.18, N 7.39.

### Reference Example 7

### Synthesis of cis-3-cyclopropylaminomethyl-4-methylpyrrolidine

### Step 1:

cis-1-Benzyl-3-hydroxy-4-methylpyrrolidine (6.81g) was dissolved in dichloromethane (70mL). While this solution was cooled on a dry ice/acetone bath, triethylamine (5.21mL) was added. Methanesulfonyl chloride (2.89mL) was then added dropwise and the mixture was further stirred for 1 hour. Following addition of water (50mL), the temperature of the mixture was allowed to rise to room temperature and the dichloromethane layer was separated. The aqueous layer was extracted with dichloromethane (50mL) and the extract was combined with the dichloromethane layer. The combined dichloromethane layers were then washed with water, followed by drying over anhydrous sodium sulfate and concentration under reduced pressure. The resulting residue was dissolved in acetonitrile (180mL). To this solution, tetrabutylammonium cyanide (23.9g) was added and the mixture was refluxed for 7 hours while heated. Subsequently, the reaction mixture was concentrated under reduced pressure and the resulting residue was dissolved in ethyl acetate (300mL). The solution was washed with water, was dried over anhydrous sodium sulfate, and was concentrated under reduced pressure. The resulting residue was purified on a silica gel column (hexane : ethyl acetate = 1:1) to give 4.61g of cis-1-benzyl-4-methyl-3-pyrrolidinecarbonitrile as a brown oil.
IR (neat): 2240, 1496, 1454 cm⁻¹.
MS (EI) m/z: 200 (M⁺).

### Step 2:

Lithium aluminum hydride (80%, 3.89g) was suspended in diethyl ether (90mL). While the suspension was cooled on an ice bath, a diethyl ether solution (25mL) of cis-1-benzyl-4-methyl-3-pyrrolidinecarbonitrile (4.11g) was added dropwise and the mixture was stirred at room temperature for 1 hour.
While the reaction mixture was cooled on an ice bath, a saturated aqueous solution of sodium hydrogen carbonate (8mL) was carefully added dropwise. Following dilution with diethyl ether (100mL), insoluble materials were collected by filtration and were washed with diethyl ether. The filtrate and the washing solution were combined and the combined solution was concentrated under reduced pressure. The resulting residue was purified on a silica gel column (hexane: ethyl acetate = 1:1 shifted to ethyl acetate: methanol = 10:1) to give 2.35g of cis-1-benzyl-4-methyl-3-aminomethylpyrrolidine as a pale yellow oil.
¹H NMR(CDCl₃) δ 0.94 (d, J = 7.3 Hz, 3H), 1.09-1.66 (br, 2H), 2.03 (dd,J = 7.3 Hz, 9.3 Hz, 1H), 2.11-2.26 (m, 2H), 2.31-2.42 (m, 1H), 2.58 (dd, J = 8_{.}3 Hz, 12.2 Hz, 1H), 2.82 (dd, J = 5.9 Hz, 12.2 Hz, 1H), 2.96-3.02 (m, 2H), 3.60 (s, 2H), 7.21-7.35 (m, 5H).

### Step 3:

cis-1-Benzyl-4-methyl-3-aminomethylpyrrolidine (1000mg) was dissolved in methanol (10mL). While this solution was cooled on an ice bath, benzaldehyde (0.50mL) was added dropwise and the mixture was stirred at room temperature for 1 hour. Subsequently, sodium cyanoborohydride (184mg) was added and the mixture was stirred at room temperature for 1.5 hours. This was followed by a second addition of sodium cyanoborohydride (123mg) and stirring for additional 5.5 hours. Subsequently, a 2mol/L aqueous solution of sodium hydroxide (5mL) was added to the reaction mixture and the mixture was refluxed for 2 hours while heated. Following concentration under reduced pressure, the resulting residue was extracted with toluene (2 x 30mL) and the toluene extracts were combined. The combined toluene layers were then washed with water, followed by drying over anhydrous sodium sulfate and concentration under reduced pressure. The resulting residue was purified on a silica gel column (hexane: ethyl acetate = 4:1) to give 690mg of cis-1-benzyl-3-benzylaminomethyl-4-methylpyrrolidine as a pale yellow oil.
MS (EI) m/z: 294 (M⁺).
HRMS (EI) : Calcd. for C₂₀H₂₆N₂ (M⁺) 294.2096; found: 294.2110.

### Step 4:

cis-1-Benzyl -3-benzylaminomethyl-4-methylpyrrolidine (680mg) was dissolved in methanol (7mL). To this solution, molecular sieves 3A (700mg), acetic acid (1.32mL), [1-(ethoxycyclopropyl)oxy]trimethylsilane (1.85mL), and sodium cyanoborohydride (435mg) were added and the mixture was refluxed for 4 hours while heated. Insoluble materials were collected by filtration and were washed with methanol. The filtrate and the washing solution were combined and the combined organic layers were concentrated under reduced pressure. To the resulting residue, water was added (5mL), followed by addition of a 2mol/L aqueous solution of sodium hydroxide to make the mixture basic. The mixture was then extracted with toluene (2 x 50mL) and the extracts were combined. The combined toluene layers were then washed with water, dried over anhydrous sodium sulfate, and then concentrated under reduced pressure. The resulting residue was purified on a silica gel column (hexane: ethyl acetate = 4:1) to give 648mg of cis-1-benzyl-3-(N-benzyl-N-cyclopropyl)aminomethyl-4-methylpyrrolidine as a colorless oil.
MS (EI) m/z: 334 (M⁺).
HRMS (EI) : Calcd. for C₂₃H₃₀N₂(M⁺) 334.2409; found: 334.2403.

### Step 5:

cis-1-Benzyl-3-(N-benzyl-N-cyclopropyl)aminomethyl-4-methylpyrrolidine (640mg) was dissolved in ethanol (10mL). To this solution, 10% palladium carbon (500mg) and chloroform (0.77mL) were added and the mixture was stirred at 50°C for 7 hours under a hydrogen pressure of 3.9 x 10⁵Pa. From the reaction mixture, the catalyst was collected by filtration and was washed with ethanol. The filtrate and the washing solution were combined and the combined organic layers were concentrated under reduced pressure. To the resulting residue, water (2mL) was added, followed by addition of a 2mol/L aqueous solution of sodium hydroxide to make the mixture basic. Sodium chloride was then added to the mixture for salting out and the mixture was extracted with diethyl ether (2 x 25mL). The diethyl ether extracts were combined and the combined diethyl ether layers were dried over anhydrous sodium sulfate and were concentrated under reduced pressure. The resulting residue was purified on a silica gel column (hexane: ethyl acetate = 4:1 shifted to dichloromethane: methanol = 10:1) to give 124mg of cis-3-cyclopropylaminomethyl-4-methylpyrrolidine as a pale brown oil.
MS (CI⁺) m/z: 155 (MH⁺).
HRMS (CI⁺): Calcd. for C₉H₁₉N₂(MH⁺): 155.1548; found: 155.1553.

### Reference Example 8

### Synthesis of (3R, 4S)-3-cyclopropylaminomethyl-4-methylpyrrolidine

### Step 1:

(3R,4S)-1-Benzyl-3-hydroxy-4-methylpyrrolidine (4.00g) was dissolved in dichloromethane (40mL). While this solution was cooled on a dry ice/acetone bath, triethylamine (3.06mL) was added. Methanesulfonyl chloride (1.70mL) was then added dropwise and the mixture was further stirred for 1 hour. Following addition of water (40mL), the temperature of the mixture was allowed to rise to room temperature and the dichloromethane layer was separated. The aqueous layer was extracted with dichloromethane (40mL) and the extract was combined with the dichloromethane layer. The combined dichloromethane layers were then washed with water, followed by drying over anhydrous sodium sulfate and concentration under reduced pressure. The resulting residue was dissolved in N,N-dimethylformamide (120mL). To this solution, tetrabutylammonium cyanide (5.53g) and sodium cyanide (2.05g) were added and the mixture was stirred at 80°C for 13 hours. Subsequently, the reaction mixture was concentrated under reduced pressure and water (50mL) was added to the resulting residue. The mixture was extracted with diethyl ether (2 x 200mL). The diethyl ether extracts were combined and the combined extracts were washed with a saturated aqueous solution of sodium chloride, followed by drying over anhydrous sodium sulfate and concentration under reduced pressure. The resulting residue was purified on a silica gel column (hexane: ethyl acetate = 4:1) to give 3.32g of (3R,4S)-1-benzyl-4-methyl-3-pyrrolidinecarbonitrile as a brown oil.
¹H NMR(CDCl₃) :δ 1.22 (d, J = 7.3 Hz, 3H), 2.12 (dd, J = 8.3 Hz, 9.3 Hz, 1H), 2.45-2.57 (m, 1H), 2.60-2.67 (m, 1H), 2.99 (dd, J = 7.3 Hz, 9.3 Hz, 1H), 3.09-3.19 (m, 2H), 3.62 (s, 2H), 7.25-7.35 (m, 5H).
MS(EI)m/z: 200 (M⁺).

### Step 2:

In a similar manner to Step 2 in Reference Example 7, (3R,4S)-1-benzyl-4-methyl-3-pyrrolidinecarbonitrile (3.20g) was reacted to obtain 2.98g of (35,45)-1-benzyl-4-methyl-3-aminomethylpyrrolidine.
¹H NMR(CDCl₃) :δ 0.94 (d, J = 7.3 Hz, 3H), 2.03 (dd, J = 7.3 Hz, 9.3 Hz, 1H), 2.11-2.26 (m, 2H), 2.31-2.43 (m, 1H), 2.58 (dd, J = 8.3 Hz, 12.2 Hz, 1H), 2.82 (dd, J = 5.9 Hz, 12.2 Hz, 1H), 2.97-3.02 (m, 2H), 3.60 (s,2H), 7.22-7.33 (m, 5H).

### Step 3:

In a similar manner to Step 3 in Reference Example 7, (3S,4S)-1-benzyl-4-methyl-3-aminomethylpyrrolidine (2.80g) was reacted to give 3.49g of (3R,4S)-1-benzyl-3-benzylaminomethyl-4-methyl-pyrrolidine.
MS (EI) m/z: 294 (M⁺).
HRMS (EI) : Calcd. for C₂₀H₂₆N₂(M⁺) 294.2096; found: 294.2072.

### Step 4:

In a similar manner to Step 4 in Reference Example 7, (3R, 4S)-1-benzyl-3-benzylaminomethyl-4-methylpyrrolidine (3.40g) was reacted to give 3.72g of ( 3R,4S)-1-benzyl-3-(N-benzyl-N-cyclopropyl) aminomethyl-4-methylpyrrolidine.
MS (FAB⁺) m/z: 335 (MH⁺).
HRMS (EI) : Calcd. for C₂₃H₃₁N₂ (MH⁺) 335.2487; found: 335.2503.

### Step 5:

In a similar manner to Step 5 in Reference Example 7, (3R,4S)-1-benzyl-3-(N-benzyl-N-cyclopropyl) aminomethy 1-4-methylpyrrolidine (3.60g) was reacted to give 1.29g of (3R,4S)-3-cyclopropylaminomethyl-4-methylpyrrolidine.
MS (CI⁺)m/z: 155 (MH⁺).
HRMS (CI⁺): Calcd. for C₉H₁₉N₂ (MH⁺): 155.1546; found: 155.1539.

### Reference Example 9

### Synthesis of (3S,4R)-3-cyclypropylaminomethyl-4-methylpyrrolidine

### Step 1:

In a similar manner to Step 1 in Example 8, (3S,4R)-1-benzyl-3-hydroxy-4-methylpyrrolidine (4.62g) was reacted to give 3.07g of (3S, 4R)=1-benzyl-4-methyl-3-pyrrolidinecarbonitrile.
¹H NMR(CDCl₃):δ 1.22 (d, J = 6.8 Hz, 3H) , 2.13 (t, J = 9.3 Hz, 1H), 2.45-2.55 (m, 1H), 2.61-2.65 (m, 1H), 2.99 (dd, J = 6.8 Hz, 9.3 Hz, 1H), 3.09-3.19 (m, 2H), 3.62 (s, 2H), 7.27-7.34 (m, 5H).

### Step 2:

In a similar manner to Step 2 in Reference Example 7, (3S,4R)-1-benzyl-4-methyl-3-pyrrolidinecarbonitrile (3.00g) was reacted to give 1.44g of (3R,4R)-1-benzyl-4-methyl-3-aminomethylpyrrolidine.
MS (EI)m/z: 204 (M⁺)
HRMS (EI) : Calcd. for C₁₃H₂₀N₂ (M⁺): 204.1626; found: 204.1614.

### Step 3:

In a similar manner to Step 3 in Reference Example 7, (3R,4R)-1-benzyl-4-methyl-3-aminomethylpyrrolidine (1.06g) was reacted to give 1.20g of (3S,4R)-1-benzyl-3-benzylaminomethyl-4-methylpyrrolidine.
MS (EI) m/z: 294 (M⁺).
HRMS (EI) : Calcd. for C₂₀H₂₆N₂(M⁺): 294.2096; found: 294.2106.

### Step 4:

In a similar manner to Step 4 in Reference Example 7, (3S,4R)-1-benzyl-3-benzylaminomethyl-4-methylpyrrolidine (1.40g) was reacted to give 1.55g of (3S,4R)-1-benzyl-3-(N-benzyl-N-cyclopropyl) aminomethyl-4-methylpyrrolidine.
MS (FAB⁺) m/z: 335 (MH⁺).
HRMS (EI): Calcd. for C₂₃H₃₁N₂ (MH⁺) : 335.2487; found: 335.2498.

### Step 5:

In a similar manner to Step 5 in Reference Example 7, (3S,4R)-1-benz-yl-3-(N-benzyl-N-cyclopropyl)aminomethyl-4-methylpyrrolidine (700mg) was reacted to give 215mg of (3S,4R)-3-cyclopropylaminomethyl-4-methylpyrrolidine.
MS (CI⁺) m/z: 155 (MH⁺)
HRMS (CI⁺): Calcd. for C₉H₁₉N₂(MH⁺): 155.1548; found: 155.1510.

### Reference Example10

### Synthesis of trans-3-cyclopropylaminomethyl-4-trifluoromethylpyrrolidine

### Step 1:

In a similar manner to Step 1 in Example 4, trans-1-benzyl-4-trifluoromethyl-3-pyrrolidinecarboxylic acid (3.00g) was reacted to give 3.32g of trans-1-benzyl-4-trifluoromethyl-3-pyrrolidinecarboxamide.
¹H HMR(CDCl₃):δ 0.42-0.46 (m, 2H), 0.75-0.79 (m, 2H), 2.64-2.78 (m, 4H), 2.82-2.86 (m, 1H), 2.95 (t, J = 9.3 Hz, 1H), 3.10-3.22 (m, 1H), 3.59 (d, J = 13.2 Hz, 1H), 3.68 (d, J = 12.7 Hz, 1H), 6.34-6.53 (br, 1H), 7.26-7.36 (m,5H).

### Step 2:

In a similar manner Step 2 in Example 4, trans-1-benzyl-4-trifluoromethyl-3-pyrrolidinecarboxamide (3.21g) was reacted to give 3.37g of trans-1-benzyl-3-[[(N-tert-butoxycarbonyl-N-cyclopropyl)amino]methyl]-4-trifluoromethylpyrrolidine.
MS (FAB⁺) m/z: 399 (MH⁺).
HRMS (FAB⁺): Calcd. for C₂₁H₃₀F₃N₂O₂ (MH⁺): 399.2259; found: 399.2254.

### Step 3:

In a similar manner to Step 3 in Example 4, trans-1-benzyl-3-[[(N-tert-butoxycarbonyl-N-cyclopropyl)amino]methyl]-4-trifluoromethylpyrrolidine (3.27g) was reacted to give 2.38g of trans-3-[[(N-tert-butoxycarbonyl-N-cyclopropyl)amino]methyl]-4-trifluoromethylpyrrolidine
MS (FAB⁺) m/z: 309 (MH⁺).
HRMS (FAB⁺): Calcd. for C₁₄H₂₄F₃N₂O₂(MH⁺): 309.1790; found: 309.1783.

### Step 4:

In a similar manner to Step 4 in Example 4, trans-3-[[(N-tert-butoxycarbonyl-N-cyclopropyl)amino]methyl]-4-trifluoromethylpyrrolidine (2.30g) was reacted to give 992mg of trans-3-cyclopropylaminomethyl-4-trifluoromethylpyrrolidine. ¹H NMR(CDCl₃): δ 0.29-0.33 (m, 2H), 0.42-0.46 (m, 2H), 2.10-2.15 (m, 1H), 2.30-2.39 (m, 1H), 2.41-2.53 (m, 1H), 2.62-2.71 (m, 2H), 2.83 (dd, J = 6.3 Hz, 11.7 Hz, 1H), 3.10 (d, J = 6.8 Hz, 2H), 3.18 (dd, J =7.8 Hz, 11.7 Hz, 1H).
Elementary analysis (%): Calcd. for C₉H₁₅F₃N₂·2CF₃COOH: C 35.79, H 3.93, N 6.42; found: C 35.82, H 3.90, N 6.59.

### Reference Example 11

### Synthesis of (3R,4S)-3-cyclopropylaminomethyl-4-fluoropyrrolidine (Process (I))

### Step 1:

(E)-3-Benzyloxypropenyl-(1R)-camphorsultam (21.6g) was dissolved in dichloromethane (300mL) containing trifluoroacetic acid (0.116mL). To this solution, N-methoxymethyl-N-(trimethylsilyl)benzylamine (15.0g) was added dropwise and the mixture was further stirred for 2 hours. The mixture was sequentially washed with a saturated aqueous solution of sodium hydrogen carbonate (2 x 200mL) and then with water (200mL), followed by drying over anhydrous sodium sulfate and concentration under reduced pressure. The resulting pale yellow oil was dissolved in diethyl ether (150mL) and the solution was allowed to stand for 18 hours at room temperature. The crystals formed were collected by filtration, washed with diethyl ether, and then dried under reduced pressure to give 11.5g of N-[[(3S,4R)-benzyl-4-benzyloxypyrrolidin-3-yl]carbonyl]-(2'S)-bornane-10,2-sultam as white crystals. The filtrate and the washing solution were combined and the combined organic layers were concentrated under reduced pressure. The resulting residue was purified on a silica gel column (eluant = cyclohexane: ethyl acetate = 4:1) to obtain additional 8.48g of N-[[(3S,4R)-benzyl-4-benzyloxypyrrolidin-3-yl]carbonyl]-(2'S)-bornane-10,2-sultam.
¹H NMR (CDCl₃): δ 0.95 (s, 3H), 1.02 (s, 3H), 1.32-1.45 (m, 2H), 1.86-1.96 (m, 3H), 2.00-2.10 (m, 2H), 2.57 (dd, J = 9.3 Hz, 5.3 Hz), 2.69 (dd, J = 9.8 Hz, 3.9 Hz, 1H), 2.93 (dd, J = 10.3 Hz, 6.3 Hz, 1H), 3.20 (t, J = 9.3Hz), 3.42-3.51 (m, 3H), 3.69-3.74 (m, 2H), 3.90 (d, J = 11.7 Hz), 4.54 (d, J = 11.7 Hz) , 4.63-4.66 (m, 1H), 7.22-7.31 (m, 10H)

### Step 2:

Lithium aluminum hydride (80%, 5.56g) was suspended in tetrahydrofuran (170mL). While the suspension was cooled on a sodium chloride/ice bath, a tetrahydrofuran solution (300mL) of N-[[(3S,4R)-benzyl-4-benzyloxypyrrolidin-3-yl]carbonyl]-(2'S)-bornane-10,2-sultam (19.9g) was added dropwise and the mixture was stirred at -5°C or below for 1 hour. Subsequently, water (34mL) was carefully added dropwise to the mixture.
Insoluble materials were collected by filtration and were washed with ethyl acetate (2 x 400mL). The filtrate and the washing solutions were combined and the combined organic layers were extracted with 1mol/L hydrochloric acid (2 x 500mL). The hydrochloric acid extracts were combined and a 30% aqueous solution of sodium hydroxide was added to make the combined solution basic (pH 14). The mixture was then extracted with diethyl ether (2 x 500mL) and the diethyl ether extracts were combined. The combined diethyl ether layers were concentrated under reduced pressure and the resulting residue was purified on a silica gel column (eluant = hexane: ethyl acetate = 1:1) to give 9.91g of (3R,4R)-(1-benzyl-4-benzyloxypyrrolidin-3-yl)methanol as a pale yellow oil.
¹H NMR(CDCl₃): δ 2.29-2.34(m, 1H), 2.40 (dd, J = 10.3 Hz, 4.4 Hz, 1H), 2.68 (dd, J = 9.3 Hz, 2.4 Hz, 1H), 2.75 (dd, J = 9.8 Hz, 6.3 Hz, 1H), 3.18 (dd, J = 9.8 Hz, 6.8 Hz, 1H), 3.61 (s, 2H), 3.65 (dd, J = 10.3 Hz, 4.4 Hz, 1H), 3.73 (dd, J = 10.3 Hz, 4.4 Hz, 1H), 4.07 (ddd, J = 6.3 Hz, 4.4 Hz, 2.0 Hz, 1H), 4.48 (s, 2H), 7.25-7.35 (m, 10H).

### Step 3:

Process (A): (3R,4R)-(1-benzyl-4-benzyloxypyrrolidine-3-yl)methanol (9.80g) was dissolved in ethanol (100mL). To this solution, 10% palladium carbon (2.00g) was added and the mixture was stirred at 50°C for 21 hours under a hydrogen pressure of 3.9 x 10⁵Pa. Subsequently, the catalyst was collected from the reaction mixture by filtration through a Celite pad. The collected catalyst and the Celite pad were washed with ethanol. The filtrate and the washing solution were combined and the combined organic layers were concentrated under reduced pressure. The resulting residue was dissolved in ethanol (100mL), followed by addition of 10% palladium carbon (2.00g). The mixture was then stirred at 50°C for 20 hours under a hydrogen pressure of 3.9 x 10⁵Pa. Subsequently, the catalyst was collected from the reaction mixture by filtration through a Celite pad. The collected catalyst and the Celite pad were washed with ethanol. The filtrate and the washing solution were combined and the combined organic layers were concentrated under reduced pressure. The resulting residue was dried under reduced pressure to give 3.77g of (3R,4R)-(4-hydroxypyrrolidin-3-yl)methanol.
¹H NMR(DMSO-d₆): δ 1.96-2.03 (m, 1H), 2.61 (dd, J = 11.6 Hz, 5.5 Hz,1H), 2.68 (dd, J = 11.6 Hz, 3.1 Hz, 1H), 2.91 (dd, J = 11.1 Hz, 5.5 Hz, 1H), 3.06 (dd, J = 11. 0 Hz, 7.3 Hz, 1H), 3.26 (dd, J = 10 .4 Hz, 7.3 Hz, 1H), 3.37 (dd, J = 10.4 Hz, 6.1 Hz), 3.90-3.93 (m, 1H).

Sodium hydroxide (2.70g) was dissolved in water (25mL) and dioxane (15mL) was added. (3R, 4R) - (4-Hydroxypyrrolidin-3-yl)methanol (1.00g) was dissolved in this solution. While the solution was cooled on an ice bath, carbobenzoxy chloride (0.97mL) was added dropwise. The mixture was stirred at 5°C or below for 1 hour, followed by dropwise addition of carbobenzoxy chloride (0.97mL). The mixture was further stirred at 5°C or below for additional 1 hour and carbobenzoxy chloride (0.97mL) was subsequently added dropwise. This was followed by stirring for 1 hour at 5°C or below and another 1 hour at room temperature. Subsequently, the reaction mixture was extracted with dichloromethane (2 x 100mL). The dichloromethane extracts were combined, and the combined dichloromethane layers were dried over anhydrous sodium sulfate and was concentrated under reduced pressure. The resulting residue was purified on a silica gel column (eluant = hexane: ethyl acetate = 1:1 shifted to ethyl acetate: methanol = 20:1) to give 1.18g of (3R,4R)-[1-benzyloxycarbonyl-4-hydroxypyrrolidin-3-yl]methanol as a milky white tar-like product.
MS (EI) m/z: 251 (M⁺).
¹H NMR(CDCl₃): δ 2.08-2.40 (br +m, 2H), 2.58-2.79 (br, 1H), 3.20 (dd, J = 11.0 Hz, 7.3 Hz, 1H), 3.32 (dt, J = 11.1Hz, 5.5 Hz, 1H), 3.59-3.76 (m, 4H), 4.23-4.33 (br, 1H), 5.12 (s, 2H), 7.28-7.36 (m, 5H).

Process (B): (3R,4R)-[1-benzyl-4-benzyloxypyrrolidin-3-yl]methanol (10.0g) was dissolved in methanol (200mL). To this solution, 10% palladium carbon (3.00g) suspended in water (60mL) and ammonium formate (21.2g) were sequentially added, and the mixture was heat-refluxed for 4 hours while being stirred. Subsequently, the catalyst was collected from the reaction mixture by filtration through a Celite pad. The collected catalyst and the Celite pad were washed with a methanol/water mixture (80:20). The filtrate and the washing solution were combined and the combined solutions were concentrated under reduced pressure. The resulting pale brown, tar-like material was dissolved in N,N-dimethylformamide (100mL). While this solution was cooled on an ice bath, triethylamine (9.40mL) was added, followed by dropwise' addition of carbobenzoxy chloride (6.00mL). While being cooled on an ice bath, the resulting mixture was stirred for 1.5 hours and was subsequently concentrated under reduced pressure. The resulting residue was dissolved in ethyl acetate (400mL) and the solution was washed with a saturated aqueous solution of sodium chloride (2 x 100mL), was dried over anhydrous sodium sulfate, and was then concentrated under reduced pressure. The resulting residue was purified on a silica gel column (eluant = ethyl acetate, shifted to ethyl acetate: methanol = 20:1) to give 7.66g of (3R,4R)-[1-benzyloxycarbonyl-4-hydroxypyrrolidin-3-yl]methanol as a milky white tar-like product.
This compound was identical to the compound obtained by

### Process (A).

### Step 4:

Process (A).: (3R,4R)-(1-benzyloxycarbonyl-4 -hydroxypyrrolidin-3-yl)methanol (3.19g) was dissolved in N,N-dimethylformamide (91mL). While this solution was cooled on an ice bath, imidazole (6.05g) and tert-butylchlorodimethylsilane (5.74g) were sequentially added and the mixture was stirred at room temperature for 3 hours. Subsequently, the reaction mixture was concentrated under reduced pressure and the resulting residue was dissolved in diethyl ether (400mL). The diethyl ether layer was washed with a saturated aqueous solution of sodium chloride (2 x 100mL), was dried over anhydrous sodium sulfate, and was then concentrated under reduced pressure. The resulting residue was purified on a silica gel column (eluant = hexane: ethyl acetate = 4:1) to give 5.46g of (3R,4R)-1-benzyloxycarbonyl-3-(tertbutyldimethylsilyl)oxymethyl-4-(tert-butyldimethylsilyl)oxypyrrolidine as a colorless oil.
MS (CI⁺): m/z = 480 (MH⁺).
¹H NMR(CDCl₃): δ 0.03 (s, 3H), 0.05 (s, 3H), 0.06 (s, 3H), 0.07 (s, 3H), 0.87 (s, 9H), 0.88 (s, 9H), 2.17-2.27 (m, 1H), 3.21-3.28 (m, 2H), 3.48-3.67 (m, 4H), 4.21-4.28 (m, 1H), 5.13 (s, 2H), 7.31-7_{.}37 (m, 5H).

(3R,4R)-1-Benzyloxycarbonyl-3-(tert-butyldimethylsilyl)oxymethyl-4-(tertbutyldimethylsilyl)oxypyrrolidine (5.46g) was dissolved in tetrahydrofuran (23mL). While this solution was cooled on an ice bath, water (23mL) and acetic acid (68mL) were sequentially added and the mixture was stirred at room temperature for 8 hours. Subsequently, the reaction mixture was concentrated under reduced pressure and the resulting residue was purified on a silica gel column (eluant = hexane: ethyl acetate = 4:1 shifted to 1:1) to give 2.74g of (3R,4R)-1-benzyloxycarbonyl-3-hydroxymethyl-4-(tert-butyldimethylsilyloxy)pyrrolidine as a colorless oil.
MS (CI⁺): m/z = 366 (MH⁺) .
¹H NMR(CDCl₃): δ 0.07-0.08 (m, 6H), 0.88 (s, 9H), 2.23-2.35 (m, 1H), 3.21-3.30 (m, 2H), 3.58-3.72 (m, 4H), 4.17-4.25 (m, 1H), 5.128 (s, 1H), 5.135 (s, 1H), 7.31-7.37 (m, 5H).

(3R,4R)-1-Benzyloxycarbonyl-3-hydroxymethyl-4-(tert-butyldimethylsilyloxy)pyrrolidine (2.73g) was dissolved in dichloromethane (60mL). While this solution was cooled on a sodium chloride/ice bath, triethylamine (1.21mL) was added, which was followed by dropwise addition of methanesulfonyl chloride (0.71mL) at -5°C or below. The reaction mixture was then stirred at -5°C or below for 1 hour, was washed with water (2 x 25mL), was dried over anhydrous sodium sulfate, and was then concentrated under reduced pressure. The resulting residue was dissolved in N,N-dimethylformamide (60mL), followed by addition of sodium azide (1.14g) and stirring at 100°C for 2 hours. The reaction mixture was then concentrated under reduced pressure and water (30mL) was added to the resulting residue. The mixture was then extracted with diethyl ether (2 x 100mL) and the diethyl ether extracts were combined. The combined diethyl ether layers were dried over anhydrous sodium sulfate and concentrated under reduced pressure. The resulting residue was purified on a silica gel column (eluant = hexane: ethyl acetate = 4:1) to give 3.06g of (3R,4R)-3-azidomethyl-1-benzyloxycarbonyl-4-(tertbutyldimethylsilyl)oxypyrrolidine as a colorless oil.
MS (CI⁺): m/z = 391 (MH⁺).
¹H NMR(CDCl₃): δ 0.07-0.09 (m, 3H), 2.23-2.34 (m, 1H), 3.19-3.25 (m, 2H), 3.27-3.40 (m, 2H), 3.60-3.71 (m, 2H), 4.11-4.17 (m, 1H), 5.13 (s, 2H), 7.31-7.37 (m, 5H).

(3R,4R)-3-Azidomethyl-1-benzyloxycarbonyl-4-(tertbutyldimethylsilyl)oxypyrrolidine (3.05g) was dissolved in tetrahydrofuran (50mL). While this solution was cooled on an ice bath, tetrabutylammonium fluoride (1mol/L tetrahydrofuran solution, 13.3mL) was added dropwise and the mixture was stirred for 1 additional hour. Subsequently, a saturated aqueous solution of sodium chloride 70mL) was added and the mixture was extracted with ethyl acetate (150mL, 100mL). The ethyl acetate extracts were combined and the combined solvents were dried over anhydrous sodium sulfate and concentrated under reduced pressure. The resulting residue was purified on a silica gel column (eluant = ethyl acetate) to give 2.01g of (3R,4R)-3-azidomethyl-1-benzyloxycarbonyl-4-hydroxypyrrolidine as a milky white syrup-like product.
MS (CI⁺): m/z = 277 (MH⁺).
¹H NMR (CDCl₃): δ 2.18-2.30 (br, 1H), 2.32-2.40 (m, 1H), 3.24 (dd, J = 11.6 Hz, 6.1 Hz, 1H), 3.30-3.47 (m, 3H), 3.68-3.75 (m, 2H), 4.18-4.24 (m, 1H), 5.13 (s, 2H), 7.31-7.37 (m, 5H).

Process (B): (3R,4R)-[1-Benzyloxycarbonyl-4-hydroxypyrrolidin-3-yl]methanol (3.00g), sodium azide (2.32g), triphenylphosphine (3.43g) and N,N-dimethylformamide (60mL) were mixed with each other. While the mixture was cooled on an ice bath, a dichloromethane solution (14mL) of carbon tetrabromide (4.34g) was added dropwise. The reaction mixture was stirred for 25 hours at room temperature and 2 additional hours at 60°C, followed by addition of methanol (5mL) and concentration under reduced pressure. The resulting residue was dissolved in ethyl acetate (200mL) and was washed with a saturated aqueous solution of sodium chloride (2 x 50mL), followed by drying over anhydrous sodium sulfate and concentration under reduce pressure. The resulting residue was purified on silica gel column (eluant = ethyl acetate: hexane = 2:1) to give 2.94g of (3R,4R)-3-azidomethyl-1-benzyloxycarbonyl-4-hydroxypyrrolidine as a pale brown syrup-like product. This compound was identical to the compound obtained by Process (A).

Process (C): (3R,4R)-[1-Benzyloxycarbonyl-4-hydroxypyrrolidin-3-yl]methanol (150mg) was dissolved in dichloromethane (12mL) and 2,4,6-collidine (0.79mL) was added. While this solution was cooled on an ice bath, methanesulfonyl chloride (46.2µL) was added dropwise. The mixture was then stirred for 2 hours on the ice bath and was allowed to stand for 15 hours in a refrigerator (3°C). Subsequently, the reaction mixture was sequentially washed with water (2mL), 1mol/L hydrochloric acid (2 x 2mL), and a saturated aqueous solution of sodium chloride (2 x 2mL), followed by drying over anhydrous sodium sulfate and concentration under reduced pressure. The resulting residue was purified on a silica gel column (eluant = hexane: ethyl acetate = 1:2 shifted to ethyl acetate) to give 38.7mg of (3R,4R)-1-benzyloxycarbonyl-3-methanesulfonyloxy-4-methanesulfonyloxymethylpyrrolidine as a pale yellow syrup-like product and 133mg of (3R,4R)-1-benzyloxycarbonyl-3-hydroxy-4-methanesulfonyloxymethylpyrrolidine as a white syrup-like product.

(3R,4R)-1-Benzyloxycarbonyl-3-hydroxy-4-methanesulfonyloxymethylpyrrolidine (.125mg) was dissolved in N,N-dimethylformamide (3mL) and sodium azide (50.0mg) was added. The mixture was stirred at 100°C for 1 hour and was then concentrated under reduced pressure. The resulting residue was dissolved in ethyl acetate (5mL) and the solution was washed with water (2 x 1mL), followed by drying over anhydrous sodium sulfate and concentration under reduced pressure. The resulting residue was purified on a silica gel column (eluant = ethyl acetate) to give 91.0mg of (3R,4R)-3 azidomethy-1-1-benzyloxycarbonyl-4-hydroxypyrrolidine as a milky white syrup-like product. The compound was identical to the compound obtained by Process (A).

### Step 5:

Process (A): (3R,4R)-3-Azidomethyl-1-benzyloxycarbonyl-4-hydroxypyrrolidine (1.20g) was dissolved in dichloromethane (40mL). While this solution was cooled on a sodium chloride/ice bath, diethylaminosulfur trifluoride (1.20mL) was added dropwise and the mixture was stirred at room temperature for 3 hours. The reaction vessel was again cooled on a sodium chloride/ice bath and diethylaminosulfur trifluoride (0.57mL) was again added dropwise. The mixture was then stirred at room temperature for 2 hours. While the reaction mixture was cooled on an ice bath, a saturated aqueous solution of sodium hydrogen carbonate (40mL) was added dropwise and the dichloromethane layer was separated. The dichloromethane layer was sequentially washed with a saturated aqueous solution of sodium hydrogen carbonate (2 x 20mL) and water (20mL), followed by drying over anhydrous sodium sulfate and concentration under reduced pressure. The resulting residue was purified on a silica gel column (eluant = hexane: ethyl acetate = 2:1) to give 726mg of (3R,4S)-3-azidomethyl-1-benzyloxycarbonyl-4-fluoropyrrolidine as a pale brown oil.
MS (CI⁺): m/z = 279 (MH⁺).
¹H NMR(CDCl₃): δ 2.34-2.54 (m, 1H), 3.22 (dt, J = 11.0 Hz, 2.4 Hz, 1H), 3.39-3.49 (m, 1H), 3.54-3.69 (m, 2H), 3.73-3.91 (m, 2H), 5.14 (s, 2H), 5.16 (dt, J = 53.2 Hz, 3.7 Hz, 1H), 7.32-7.37 (m, 5H).

Process (B): (3R,4R)-3-Azidomethyl-1-benzyloxycarbonyl-4-hydroxypyrrolidine (1.79g) was dissolved in toluene (56mL).
While this solution was cooled on an ice bath, 1,8-diazabicyclo[5.4.0]undec-7-ene (2.03mL) was added. This was followed by dropwise addition of perfluoro-1-octanesulfonyl fluoride (2.80mL) and stirring for another 1 hour. Insoluble materials were removed from the reaction mixture by filtration and were washed with toluene. The filtrate and the washing solution were combined and the combined organic layers were concentrated under reduced pressure. The resulting residue was then purified on a silica gel column (eluant = hexane: ethyl acetate = 2:1) to give 1.58g of (3R,4S)-3-azidomethyl-1-benzyloxycarbonyl-4-fluoropyrrolidine as a pale brown syrup-like product. The compound was identical to the compound obtained by Process (A).

### Step 6:

(3R,4S)-3-Azidomethyl-1-benzyloxycarbonyl-4-fluoropyrrolidine (1.35g) was dissolved in ethanol (30mL). To this solution, platinum oxide (IV) (190mg) was added and the mixture was stirred at room temperature for 2 hours in a stream of hydrogen (provided from a balloon). Subsequently, the catalyst was collected from the reaction mixture by filtration through a Celite pad. The collected catalyst and the Celite pad were washed with ethanol. The filtrate and the washing solution were combined and the combined organic layers were concentrated under reduced pressure. The resulting residue was purified on a silica gel column (eluant = ethyl acetate: methanol = 10:1) to give 1.13g of (3S,4S)-3-aminomethyl-1-benzyloxycarbonyl-4-fluoropyrrolidine as a pale brown oil.
MS (CI⁺): m/z = 253 (MH⁺).

### Step 7:

(3S,4S)-3-Aminomethyl-1-benzyloxycarbonyl-4-fluoropyrrolidine (1.10g) was dissolved in methanol (13mL). To this solution, molecular sieves 4A (440mg) and benzaldehyde (0.44mL) were sequentially added and the mixture was stirred at room temperature for 1 hour. Subsequently, a borane-pyridine complex (0.44mL) was added and the mixture was further stirred at room temperature for 3.5 hours. This was followed by addition of 6mol/L hydrochloric acid (7.3mL) and stirring at room temperature for 1 hour. Subsequently, a 30% aqueous solution of sodium hydroxide was added to make the mixture basic and the mixture was extracted with diethyl ether (2 x 100mL). The diethyl ether extracts were combined and the combined diethyl ether layers were dried over anhydrous sodium sulfate and then concentrated under reduced pressure. The resulting residue was purified on a silica gel column (eluant = hexane: ethyl acetate = 4:1 shifted to 1:1) to give 1.18g of (3S,4S)-3-benzylaminomethyl-1-benzyloxycarbonyl-4-fluoropyrrolidine as a colorless tar-like product.
MS (CI⁺): m/z = 343 (MH⁺).

### Step 8:

(3S,4S)-3-Benzylaminomethyl-1-benzyloxycarbonyl-4-fluoropyrrolidine (1.15g) was dissolved in methanol (21mL). To this solution, molecular sieves 3A (1.05g), acetic acid (1.92mL), [(1-ethoxycyclopropyl)oxy]trimethylsilane (2.70mL), and sodium cyanoborohydride (633mg) were added and the mixture was heat-refluxed for 2 hours while being stirred. Subsequently, insoluble materials were removed from the reaction mixture by filtration through a Celite pad. The insoluble materials and the Celite pad were washed with methanol. The filtrate and the washing solution were combined and a 2mol/L aqueous solution of sodium hydroxide was added to make the combined organic layer basic (pH14). Methanol was then removed under reduced pressure and the residue was extracted with diethyl ether (2 x 100mL). The diethyl ether extracts were combined and the combined diethyl ether layers were dried over anhydrous sodium sulfate and then concentrated under reduced pressure. The resulting residue was purified on a silica gel column (eluant = hexane: ethyl acetate = 4:1) to give 1.26g of (3S,4S)-3-(N-benzyl-N-cyclopropyl)aminomethyl-1-benzyloxycarbonyl-4-fluoropyrrolidine as a colorless tar-like product.
MS (EI) m/z: = 382 (M⁺).

### Step 9:

(3S,4S)-3-(N-Benzyl-N-cyclopropyl)aminomethyl-1-benzyloxycarbonyl-4-fluoropyrrolidine (1.22g) was dissolved in ethanol (14mL). To this solution, 10% palladium carbon (150mg) was added and the mixture was stirred at room temperature for 4 hours in a stream of hydrogen (provided from a balloon). Subsequently, the catalyst was collected from the reaction mixture by filtration through a Celite pad. The collected catalyst and the Celite pad were washed with ethanol. The filtrate and the washing solution were combined and the combined organic layers were concentrated under reduced pressure. The resulting residue was purified on a silica gel column (eluant = ethyl acetate: methanol = 20:1). The eluate was distilled under reduced pressure to give 414mg of (3R,4S)-3-cyclopropylaminomethyl-4-fluoropyrrolidine as a colorless oil.
MS (CI⁺): m/z = 159 (MH⁺).
HRMS (CI⁺): Calcd. for C₈H₁₆FN₂: 159.1298; found: 159.1316.

### Reference Example 12

### Synthesis of (3R,4S)-3-cyclopropylaminomethyl-4-fluoropyrrolidine (Process (II))

### Step 1:

(3R,4R)-(4-Hydroxypyrrolidin-3-yl)methanol (1.18g) was dissolved in ethanol (25mL) and triethylamine (1.40mL) was added to the solution. While this mixture was cooled on a sodium chloride/ice bath, benzyl bromide (1.10mL) was added dropwise. The mixture was then stirred at room temperature for 1 hour and was concentrated under reduced pressure. The resulting residue was purified on a silica gel column (eluant = ethyl acetate: methanol = 20:1) to give 1.02g of (3R,4R)-(1-benzyl-4-hydroxypyrrolidin-3-yl)methanol as a milky white syrup-like product.
MS (EI⁺): m/z = 207 (M⁺).
HRMS (EI⁺): Calcd. for C₁₂H₁₇NO₂: 207.1259; found: 207.1237.

### Step 2:

(3R,4R)-(1-Benzyl-4-hydroxypyrrolidin-3-yl)methanol (1.36g) was dissolved in dichloromethane (14mL). While this solution was cooled on an dry ice/acetone bath, triethylamine (0.83mL) was added, followed by dropwise addition of methanesulfonyl chloride (0.46mL) and stirring for 30min.
Water (10mL) was then added to the reaction mixture and the temperature of the mixture was allowed to rise to room temperature. The mixture was then diluted with dichloromethane (20mL) and the dichloromethane layer was collected. The collected dichloromethane layers were washed with water (2 x 10mL), dried over anhydrous sodium sulfate, and then concentrated under reduced pressure. The resulting residue was purified on a silica gel column (hexane: ethyl acetate = 1:1 shifted to ethyl acetate: methanol = 20:1). From a fraction eluted at hexane: ethyl acetate = 1:1, 585mg of (3R,4R)-1-benzyl-3-methanesulfonyloxy-4-methanesulfonyloxymethylpyrrolidine was obtained as a milky white syrup-like product.
MS (EI⁺): m/z = 363 (M⁺).
HRMS (EI⁺) : Calcd. for C₁₄H₂₁NO₆S₂: 363.0810; found: 363.0804.

Also, 840mg of (3R, 4R)-1-benzyl-3-hydroxy-4-methanesulfonyloxymethylpyrrolidine was obtained as white crystals from a fraction eluted at ethyl acetate: methanol = 20:1. MS (EI⁺) : m/z = 285 (M⁺).
HRMS (EI⁺) : Calcd. for C₁₃H₁₉NO₄S: 285.1035; found: 285.1045.

### Step 3:

(3R, 4R)-1-Benzyl-3-hydroxy-4-methanesulfonyloxymethylpyrrolidine (835mg), sodium azide (381mg), and N,N-dimethylformamide (12mL) were mixed with one another and the mixture was stirred at 120°C for 1 hour. Subsequently, the reaction mixture was concentrated under reduced pressure. To the resulting residue, water (10mL) was added and the mixture was extracted with diethyl ether (2 x 30mL). The diethyl ether extracts were combined and the combined extracts were dried over anhydrous sodium sulfate and concentrated under reduced pressure. The resulting residue was purified on a silica gel column (eluant = ethyl acetate: methanol = 20:1) to give 576mg of (3R,4R)-3-azidomethyl-1-benzyl-4-hydroxypyrrolidine as a pale brown oil.
MS (EI⁺): m/z = 232 (M⁺).
HRMS (EI⁺): Calcd. for C₁₂H₁₆N₄O: 232.1324; found: 232.1309.

### Step 4:

(3R,4R)-3-Azidomethyl-1-benzyl-4-hydroxypyrrolidine (566mg) was dissolved in dichloromethane (9mL). While this solution was cooled on an ice bath, diethylaminosulfur trifluoride (0.39mL) was added dropwise and the mixture was stirred at room temperature for 2 hours. While the reaction vessel was cooled on an ice bath, a saturated aqueous solution of sodium hydrogen carbonate (9mL) was added, and the mixture was diluted with dichloromethane (15mL). The dichloromethane layers were collected and the collected dichloromethane layers were washed with a saturated aqueous solution of sodium hydrogen carbonate (10mL) and then water (10mL), were dried over anhydrous sodium sulfate, and were concentrated under reduced pressure. The resulting residue was purified on a silica gel column (eluant = hexane: ethyl acetate = 4:1). From the first half fraction, 76.7mg of (3R,4R)-3-azidomethyl-1-benzyl-4-fluoropyrrolidine was obtained as a pale brown oil.
MS (EI⁺): m/z = 234 (M⁺).
HRMS (EI⁺): Calcd. for C₁₂H₁₅FN₄: 234.1281; found: 234.1263.

From the second half fraction, 220mg of (3R,4S)-3-azidomethyl-1-benzyl-4-fluoropyrrolidine was obtained as a pale brown oil.
MS (EI⁺): m/z = 234 (M⁺).
HRMS (EI⁺): Calcd. for C₁₂H₁₅FN₄: 234.1281; found: 234.1269.

### Step 5:

(3R,4S)-3-Azidomethyl-1-benzyl-4-fluoropyrrolidine (215mg) was dissolved in ethanol (3mL). To this solution, platinum oxide (IV) (30.0mg) was added and the mixture was stirred at room temperature for 5 hours in a stream of hydrogen (provided from a balloon). Subsequently, the catalyst was removed from the reaction mixture by filtration through a Celite pad. The removed catalyst and the Celite pad were washed with ethanol. The filtrate and the washing solution were combined and the combined organic layers were concentrated under reduced pressure to obtain 191mg of (3S,4S)-3-aminomethyl-1-benzyl-4-fluoropyrrolidine as a brown oil.
MS (CI⁺): m/z = 209 (MH⁺).
HRMS (CI⁺): Calcd. for C₁₂H₁₈FN₂: 209.1454; found: 209.1465.

### Step 6:

(3S,4S)-3-Aminomethyl-1-benzyl-4-fluoropyrrolidine (186mg) was dissolved in methanol (4mL). To this solution, molecular sieves 4A (80.0mg) and benzaldehyde (90.8µL) were sequentially added and the mixture was stirred at room temperature for 1 hour. Subsequently, a borane-pyridine complex (90.2µL) was added and the mixture was further stirred at room temperature for 3 hours. This was followed by addition of 6mol/L hydrochloric acid (1.5mL) and stirring for 1 hour. Subsequently, a 6mol/L aqueous solution of sodium hydroxide was added to make the mixture basic and the mixture was extracted with diethyl ether (3 x 10mL). The diethyl ether extracts were combined and the combined diethyl ether layers were dried over anhydrous sodium sulfate and then concentrated under reduced pressure. The resulting residue was purified on a silica gel column (eluant = hexane: ethyl acetate = 4:1) to give 179mg of (3S,4S)-1-benzyl-3-benzylaminomethyl-4-fluoropyrrolidine as a pale brown oil.
MS (CI⁺): m/z = 299 (MH⁺).
HRMS (CI⁺): Calcd. for C₁₉H₂₄FN₂: 299.1924; found: 299.1960.

### Step 7:

(3S,4S)-1-Benzyl-3-benzylaminomethyl-4-fluoropyrrolidine (175mg) was dissolved in methanol (2mL). To this solution, molecular sieves 3A (180mg), acetic acid (0.36mL), [(1-ethoxycyclopropyl)oxy]trimethylsilane (0.47mL) and sodium cyanoborohydride (110mg) were added and the mixture was heat-refluxed for 3 hours while being stirred. Subsequently, insoluble materials were removed from the reaction mixture by filtration through a Celite pad. The insoluble materials and the Celite pad were washed with methanol. The filtrate and the washing solution were combined and a 2mol/L aqueous solution of sodium hydroxide was added to make the combined organic layers basic (pH14). Methanol was then removed under reduced pressure and the residue was extracted with diethyl ether (3 x 100mL). The diethyl ether extracts were combined and the combined diethyl ether layers were dried over anhydrous sodium sulfate and then concentrated under reduced pressure. The resulting residue was purified on a silica gel column (eluant = hexane: ethyl acetate = 4:1) to give 172mg of (3R,4S)-3-(N-benzyl-N-cyclopropyl)aminomethyl-1-benzyl-4-fluoropyrrolidine as a colorless tar-like product.
MS (CI⁺): m/z = 339 (MH⁺).
HRMS (CI⁺): Calcd. for C₂₂H₂₈FN₂: 339.2237; found: 339.2285.

### Step 8:

(3R,4S)-3-(N-Benzyl-N-cyclopropyl)aminomethyl-1-benzyl-4-fluoropyrrolidine (170mg) was dissolved in ethanol (10mL). To this solution, 10% palladium carbon (200mg) and chloroform (0.17mL) were added and the mixture was stirred at 50°C for 23 hours under a hydrogen pressure of 3.9x10⁵Pa. Subsequently, palladium carbon was removed from the reaction mixture by filtration through a Celite pad. The removed palladium carbon and the Celite pad were washed with ethanol. The filtrate and the washing solution were then combined and the combined organic layers were concentrated under reduced pressure. To the resulting residue, a 30% aqueous solution of sodium hydroxide (approximately 1mL) was added. Subsequently, sodium chloride was added to saturation and the mixture was extracted with diethyl ether (3 x 10mL). The diethyl ether extracts were combined and the combined diethyl ether layers were dried over anhydrous sodium sulfate and then concentrated under reduced pressure to give 65.4mg of (3R,4S)-3-cyclopropylaminomethyl-4-fluoropyrrolidine as a pale brown oil.
This compound was identical to the compound obtained in Reference Example 11 (Process (I)).

### Reference Example 13

### Synthesis of (3R,4R)-3-cyclopropylaminomethyl-4-fluoropyrrolidine

### Step 1:

(3R,4R)-[1-Benzyloxycarbonyl-4-hydroxypyrrolidin-3-yl]methanol (2.50g), triphenylphosphine (5.74g), and benzoic acid (2.55g) were dissolved in tetrahydrofuran (60mL). While this solution was cooled on a sodium chloride/ice bath, azodicarboxylic acid diethyl ester (40% toluene solution, 9.53mL) was added dropwise. The mixture was stirred for 1 hour at 0°C or below and then for 2 additional hours at room temperature and was subsequently concentrated under reduced pressure. The resulting residue was purified on a silica gel column (eluant = hexane: ethyl acetate = 2:1). The eluted pale brown tar-like material was dissolved in ethanol (60mL). To this solution, potassium carbonate (4.07g) dissolved in water (30mL) was added and the mixture was heat-refluxed for 3 hours while being stirred. Subsequently, the reaction mixture was concentrated under reduced pressure, and the resulting residue was dissolved in dichloromethane (200mL). The dichloromethane solution was washed with a saturated aqueous solution of sodium chloride (2 x 50mL), was dried over anhydrous sodium sulfate, and was then concentrated under reduced pressure. The resulting residue was purified on a silica gel column (eluant = ethyl acetate: methanol = 10:1) to give 2.04g of (3R,4S)-[1-benzyloxycarbonyl-4-hydroxypyrrolidin-3-yl]methanol as a milky white syrup-like product.
MS (EI) : m/z = 251 (M⁺).

### Step 2:

(3R,4S)-[1-Benzyloxycarbonyl-4-hydroxypyrrolidin-3-yl]methanol (2.33g), sodium azide (1.81g), triphenylphosphine (2.67g), and N,N-dimethylformamide (46mL) were mixed with one another. While this mixture was cooled on an ice bath, a dichloromethane solution (10mL) of carbon tetrabromide (3.38g) was added dropwise. The reaction mixture was stirred for 13 hours at room temperature and 3 additional hours at 60°C, followed by addition of methanol (3mL) and concentration under reduced pressure. The resulting residue was dissolved in ethyl acetate (200mL) and was washed with a saturated aqueous solution of sodium chloride (2 x 50mL), followed by drying over anhydrous sodium sulfate and concentration under reduce pressure. The resulting residue was purified on a silica gel column (eluant = ethyl acetate: hexane = 2:1) to give 2.18g of (3R,4S)-3-azidomethyl-1-benzyloxycarbonyl-4-hydroxypyrrolidine as a milky white syrup-like product.
MS (FAB⁺): m/z = 277 (MH⁺).

### Step 3:

(3R,4S)-3-Azidomethyl-1-benzyloycarbonyl-4-hydroxypyrrolidine (300mg) was dissolved in dichloromethane (6mL). While this solution was cooled on an ice bath, diethylaminosulfur trifluoride (0.43mL) was added dropwise.
The mixture was stirred at room temperature for 4 hours. While the reaction vessel was cooled on an ice bath, a saturated aqueous solution of sodium hydrogen carbonate (6mL) was added and the dichloromethane layer was collected. The collected dichloromethane layer was washed with a saturated aqueous solution of sodium chloride (2 x 2mL), was dried over anhydrous sodium sulfate, and was then concentrated under reduced pressure. The resulting residue was purified on a silica gel column (eluant = hexane: ethyl acetate = 2:1) to give 211mg of a mixture of (3R, 4R)-3-azidomethyl-1-benzyloxycarbonyl-4-fluoropyrrolidine and 3-azidomethyl-1-benzyloxycarbonyl-3-pyrroline.

### Step 4:

Platinum oxide (IV) (50.0mg) was suspended in ethanol (7mL) and the suspension was stirred at room temperature for 30min in a stream of hydrogen (provided from a balloon). To this suspension, an ethanol solution (3mL) of a mixture (551mg) of (3R,4R)-3-azidomethyl-1-benzyloxycarbonyl-4-fluoropyrrolidine and 3-azidomethyl-1-benzyloxycarbonyl-3-pyrroline was added, and the mixture was stirred at room temperature for 5 hours in a stream of hydrogen (provided from a balloon). Subsequently, the catalyst was removed from the reaction mixture by filtration and was washed with ethanol. The filtrate and the washing solution were combined and the combined organic layers were concentrated under reduced pressure.
The resulting residue was then purified on a silica gel column (eluant = ethyl acetate shifted to ethyl acetate: methanol = 10:1) to give 313mg of a mixture of (3S,4R)-3-aminomethyl-1-benzyloxycarbonyl-4-fluoropyrrolidine and 3-aminomethyl-1-benzyloxycarbonyl-3-pyrroline.

### Step 5:

A mixture (310mg) of (3S,4R)-3-aminomethyl-1-benzyloxycarbonyl-4-fluoropyrrolidine and 3-aminomethyl-1-benzyloxycarbonyl-3-pyrroline was dissolved in methanol (4mL). To this solution, molecular sieves 4A (130mg) and benzaldehyde (0.13mL) were sequentially added and the mixture was stirred at room temperature for 1 hour. Subsequently, a borane-pyridine complex (0.19mL) was added and the mixture was further stirred at room temperature for 4 hours. This was followed by addition of 6mol/L hydrochloric acid (2mL) and strirring at room temperature for 1 hour. Subsequently, a 30% aqueous solution of sodium hydroxide was added to make the mixture basic and the mixture was extracted with diethyl ether (3x 10mL). The diethyl ether extracts were combined and the combined diethyl ether layers were concentrated under reduced pressure. The resulting residue was purified on a silica gel column (eluant = dichloromethane methanol = 10:1) to give 177mg of (3S,4R)-3-benzylaminomethyl-1-benzyloxycarbonyl-4-fluoropyrrelidine as a pale yellow oil.
MS (FAB⁺): m/z =343 (MH⁺).
HRMS (FAB⁺): Calcd. for C₂₀H₂₄FN₂O₂: 343.1822; found: 343.1815.

### Step 6:

(3S,4R)-3-Benzylaminomethyl-1-benzyloxycarbonyl-4-fluoropyrrolidine (170mg) was dissolved in methanol (5mL). To this solution, molecular sieves 3A (160mg), acetic acid (0.29mL), [(1-ethoxycyclopropyl)oxy]trimethylsilane (0.40mL), and sodium cyanoborohydride (93.5mg) were added and the mixture was heat-refluxed for 3 hours while being stirred. Subsequently, insoluble materials were removed from the reaction mixture by filtration through a Celite pad. The insoluble materials and the Celite pad were washed with methanol. The filtrate and the washing solution were combined and a 2mol/L aqueous solution of sodium hydroxide was added to make the combined organic layers basic (pH>12). Methanol was then removed under reduced pressure and the residue was extracted with diethyl ether (3 x 10mL). The diethyl ether extracts were combined and the combined diethyl ether layers were dried over anhydrous sodium sulfate and then concentrated under reduced pressure. The resulting residue was purified on a silica gel column (eluant = hexane: ethyl acetate = 2:1) to give 166mg of (3S,4R)-3-(N-benzyl-N-cyclopropyl)aminomethyl-1-benzyloxycarbonyl-4-fluoropyrrolidine as a colorless tar-like product.
MS (FAB⁺): m/z = 383 (MH⁺).
HRMS (FAB⁺): Calcd. for C₂₃H₂₈FN₂O₂:383.2135; found:383.2119.

### Step 7:

(3S,4R)-3-(N-Benzyl-N-cyclopropyl)aminomethyl-1-benzyloxycarbonyl-4-fluoropyrrolidine (160mg) was dissolved in ethanol (3mL). To this solution, 10% palladium carbon (20.0mg) was added and the mixture was stirred at room temperature for 5 hours in a stream of hydrogen (provided from a balloon). Subsequently, the catalyst was collected from the reaction mixture by filtration through a Celite pad. The collected catalyst and the Celite pad were washed with ethanol. The filtrate and the washing solution were combined and the resulting residue was purified on a silica gel column (eluant = ethyl acetate: methanol = 20:1, shifted to dichloromethane: methanol = 10:1) to give 50.7mg of (3R,4R)-3-cyclopropylaminomethyl-4-fluoropyrrolidine as a colorless oil.
MS (FAB⁺): m/z = 159 (MH⁺).
HRMS (FAB⁺): Calcd. for C₈H₁₆FN₂: 159.1298; found: 159.1286.

### Reference Example 14

### Synthesis of (3R,4S)-3-[(N-tert-butoxycarbonyl-N-cyclopropyl) amino]methyl-4-fluoromethylpyrrolidine

### Step 1:

(1S,5R)-7-[(1R)-1-Phenylethyl]-3-oxa-7-azabicyclo[3.3.0]octane-2-one (7.73g, 33.4mmol) was dissolved in ethanol (92mL). To this solution, cyclopropylamine (46.3ml) was added, and the mixture was stirred at 80°C for 44 hours and was subsequently concentrated under reduced pressure. The resulting residue was dissolved in ethyl acetate (300mL) and the solution was washed with water (2 x 50mL), followed by drying over anhydrous sodium sulfate and concentration under reduced pressure. To the resulting residue, diisopropyl ether (300mL) was added and the mixture was heated to form crystals and was then concentrated to approximately 1/2. The formed crystals were collected by filtration and the collected crystals were washed with diisopropyl ether and dried under reduced pressure to give 4.41g of (3R,4S)-N-cyclopropyl-4-hydroxymethyl-1-[(1S)-1-phenylethyl]pyrrolidine-3-carboxamide as white crystals. The filtrate and the washing solution were combined and the combined solvents were concentrated under reduced pressure. The resulting residue was purified on a silica gel column (eluant = hexane: ethyl acetate = 1:1, shifted to ethyl acetate) to obtain additional 1.50g of - (3R,4S)-N-cyclopropyl-4-hydroxymethyl-1-[(1S)-1-phenylethyl]pyrrolidine-3-carboxamide. The total amount of the compound was 5.91g.
MS (EI): m/z = 288 (M⁺).
Elementary analysis (%): Calcd. for C₁₇H₂₄N₂O₂·0.2H₂O: C 69.93, H 8.42, N 9.59; found: C 70.16, H 8.32, N 9.60.

### Step 2:

(3R,4S)-N-Cyclopropyl-4-hydroxymethyl-1-[(1S)-1-phenylethyl]pyrrolidine-3-carboxamide (7.54g) was dissolved in N,N-dimethylformamide (180mL). While this solution was cooled on an ice bath, imidazole (2.67g) and tert-butylchlorodimethylsilane (4.72g) were sequentially added. The mixture was stirred at room temperature for 90min and was subsequently concentrated under reduced pressure. The resulting residue was dissolved in ethyl acetate (300mL) and the solution was washed with water (2 x 100mL), followed by drying over anhydrous sodium sulfate and concentration under reduced pressure. The resulting residue was purified on a silica gel column (eluant = ethyl acetate) to give 7.05g of (3R,4S)-N-cyclopropyl-4-(tert-butyldimethylsilyl)oxymethyl-1-[(1S)-1-phenylethyl]pyrrolidine-3-carboxamide as a pale yellow tar-like product.
MS (EI) m/z: = 402 (M⁺).

### Step 3:

(3R,4S)-N-Cyclopropyl-4-(tertbutyldimethylsilyl)oxymethyl-1-[(1S)-1-phenylethyl]pyrrolidine-3-carboxamide (7.00g) was dissolved in toluene (70mL). To this solution, borane-dimethyl sulfide complex (2.20mL) was added and the mixture was heat-refluxed for 5 hours while being stirred. Subsequently, the reaction mixture was allowed to cool to room temperature. Following addition of a 10% aqueous solution of sodium carbonate (42mL), the mixture was stirred at 100°C for 1 hour and the toluene layer was separated. The toluene layer was washed with water (2 x 30mL), was dried over anhydrous sodium sulfate, and was then concentrated under reduced pressure. The resulting residue was purified on a silica gel column (eluant = hexane: ethyl acetate = 4:1) to give 4.78g of (3S,4S)-4-(tertbutyldimethylsilyl)oxymethyl-3-cyclopropylaminomethyl-1-[(1S)-1-phenylethyl]pyrrolidine as a colorless oil.

### Step 4:

(3S,4S)-4-(tert-Butyldimethylsilyl)oxymethyl-3-cyclopropylaminomethyl-1-[(1S)-1-phenylethyl]pyrrolidine (4.70g) was dissolved in dichloromethane (70mL). To this solution, di-tert-butyldicarbonate (2.77g) was added and the mixture was stirred at room temperature for 2 hours.

Subsequently, the reaction mixture was concentrated under reduced pressure and the resulting residue was purified on a silica gel column (eluant = hexane: ethyl acetate = 4:1, shifted to 1:1) to give 5.28g of (3R,4S)-3-[(N-tert-butoxycarbonyl-N-cyclopropyl)amino]methyl-4-(tertbutyldimethylsilyl)oxymethyl-1-[(1S)-1-phenylethyl]pyrrolidine as a colorless oil.

### Step 5:

Process (A): (3R,4S)-N-Cyclopropyl-4-hydroxymethyl-1-[(1S)-1-phenylethyl]pyrrolidine-3-carboxamide (1.49g) was dissolved in toluene (15mL). To this solution, a borane-dimethyl sulfide complex (0.65mL) was added and the mixture was heat-refluxed for 6 hours while being stirred. Subsequently, the reaction mixture was allowed to cool to room temperature. Following addition of a 10% aqueous solution of sodium carbonate (12.4mL), the mixture was stirred at 100°C for 1 hour and the toluene layer was separated. The toluene layer was then washed with water (10mL) and was dried over anhydrous sodium sulfate. Following addition of di-tert-butyldicarbonate (1.13g), the mixture was stirred at room temperature for 30min and was subsequently allowed to stand overnight. The reaction mixture was then concentrated under reduced pressure and the resulting residue was purified on a silica gel column (eluant = hexane: ethyl acetate = 1:1) to give 1.50g of (3R,4S)-3-[(N-tert-butoxycarbonyl-N-cyclopropyl)amino]methyl-4-hydroxymethyl-1-[(1S)-1-phenylethyl]pyrrolidine as pale brown crystals.

Process (B): (3R,4S)-3-[(N-tert-Butoxycarbonyl-N-cyclopropyl)amino]methyl-4-(tert-butyldimethylsilyl)oxymethyl-1-[(1S)-1-phenylethyl]pyrrolidine (3.02g) was dissolved in tetrahydrofuran (45mL). While this solution was cooled on an ice bath, tetrabutylammonium fluoride (1mol/L tetrahydrofuran solution, 7.42ml) was added dropwise and the mixture was stirred at room temperature for 2 hours. Subsequently, a saturated aqueous solution of sodium chloride (60mL) was added and the mixture was extracted with ethyl acetate (2 x 150mL). The ethyl acetate extracts were combined and the combined ethyl acetate layers were washed with a saturated aqueous solution of sodium chloride (2 x 100mL), followed by drying over anhydrous sodium sulfate and concentration under reduced pressure. The resulting residue was dissolved in ethyl acetate (10mL) and the formed crystals were collected by filtration, washed with a small amount of ethyl acetate, and then dried under reduced pressure to give 781mg of (3R,4S)-3-[(N-tert-butoxycarbonyl-N-cyclopropyl)amino]methyl-4-hydroxymethyl-1-[(1S)-1-phenylethyl]pyrrolidine as white crystals. The filtrate and the washing solution were combined and the combined organic layers were concentrated under reduced pressure. The resulting residue was purified on a silica gel column (eluant = hexane: ethyl acetate = 1:1) to give additional 1.43g of (3R,4S)-3-[(N-tert-butoxycarbonyl-N-cyclopropyl)amino]methyl-4-hydroxymethyl-1-[(1S)-1-phenylethyl]pyrrolidine. The total amount of the compound was 2.21g.
MS (EI) m/z: = 374 (M⁺).
Elementary analysis (%): Calcd. for C₂₂H₃₄N₂O₃: C 70.55, H 9.15, N 7.48; found: C 70.56, H 9.29, N 7.52.

### Step 6:

(3R,4S)-3-[(N-tert-Butoxycarbonyl-N-cyclopropyl)amino]methyl-4-hydroxymethyl-1-[(1S)-1-phenylethyl]pyrrolidine (2.66g) was dissolved in dichloromethane (40mL). While this solution was cooled on a sodium chloride/ice bath, triethylamine (1.05mL) was added. This was followed by dropwise addition of methanesulfonyl chloride (0.58mL). After being stirred at -5°C for 30min, the reaction mixture was washed with water, was dried over anhydrous sodium sulfate, and was then concentrated under reduced pressure. The resulting residue was dissolved in tetrahydrofuran (21mL). To this solution, tetrabutylammonium fluoride (1mol/L tetrahydrofuran solution, 21.3mL) was added and the mixture was heat-refluxed for 2 hours while being stirred. The reaction mixture was concentrated under reduced pressure and the resulting residue was dissolved in ethyl acetate (200mL). The ethyl acetate solution was washed with water (2 x 50mL), was dried over anhydrous sodium sulfate, and was then concentrated under reduced pressure. The resulting residue was purified on a silica gel column (eluant = hexane: ethyl acetate = 4:1, shifted to 1:1) to give 1.13g of (3R,4S)-3-[(N-tert-butoxycarbonyl-N-cyclopropyl)amino]methyl-4-fluoromethyl-1-[(1S)-1-phenylethyl]pyrrolidine as a pale brown tar-like product.
MS (EI) m/z = 376 (M⁺).

### Step 7:

(3R,4S)-3-[(N-tert-Butoxycarbonyl-N-cyclopropyl)amino]methyl-4-fluoromethyl-1-[(1S)-1-phenylethyl]pyrrolidine (1.10g) was dissolved in methanol (20mL). To this solution, a suspension of 10% palladium carbon (230mg) in water (4mL) and ammonium formate (921mg) were sequentially added and the mixture was heat-refluxed for 90min while being stirred. Subsequently, the catalyst was removed from the reaction mixture by filtration through a Celite pad. The removed catalyst and the Celite pad were washed with ethanol containing 20% water. The filtrate and the washing solution were combined and the combined solutions were concentrated under reduced pressure. Water (20mL) was then added to the resulting residue. While this mixture was cooled on an ice bath, a 30% aqueous solution of sodium hydroxide was added to make the mixture basic (pH14). The mixture was subsequently extracted with dichloromethane (50mL x 2). The dichloromethane extracts were combined, washed with water (2 x 20mL), and the combined dichloromethane layers were dried over anhydrous sodium sulfate and then concentrated under reduced pressure. The resulting residue was purified on a silica gel column (eluant = dichloromethane: methanol = 20:1) to give 684mg of (3R,4S)-3-[(N-tert-butoxycarbonyl-N-cyclopropyl)amino]methyl-4-fluoromethylpyrrolidine as a pale brown tar-like product.
MS (EI) m/z = 272 (M⁺) .

### Reference Example 15

### Synthesis of (3R,4R)-3-cyclopropylaminomethyl-4-methylpyrrolidine·trifluoroacetate

### Step 1:

1-Benzyl-4-(R)-methyl-3-(R)-[(4-(S)-phenyl-2-oxazolidinone-3-yl)carbonyl]pyrrolidine (150g) was dissolved in cyclopropylamine (650mL). The mixture was stirred at room temperature for 23 hours and was subsequently concentrated under reduced pressure. To the resulting residue, diisopropyl ether (800mL) was added and the mixture was stirred at room temperature for 70min. The resulting crystals were then collected by filtration. The collected crystals were then dissolved in dichloromethane (800mL) and the solution was extracted with 1mol/L hydrochloric acid (2 x 400mL). The 1mol/L hydrochloric acid extracts were combined. While the combined solutions were cooled on an ice bath, a 30% aqueous solution of sodium hydroxide was added to make the solution basic (pH13). The resulting crystals were collected by filtration, sequentially washed with water and diisopropyl ether, and then dried under reduced pressure to give 52.2g of (3R,4R)-1-1-benzyl-N-cyclopropyl-4-methyl-3-pyrrolidinecarboxamide as white crystals.

### Step 2:

(3R,4R)-1-Benzyl-N-cyclopropyl-4-methyl-3-pyrrolidinecarboxamide (70.0g) was dissolved in toluene (700mL). While this solution was cooled on an ice bath, a borane-dimethyl sulfide complex (90%, 34.3mL) was added dropwise. The mixture was then stirred for 15min and was heat-refluxed. After the reaction mixture was cooled to room temperature, a 10% aqueous solution of Na₂CO₃ (400mL) was added and the mixture was stirred at 100°C for 2 hours. The mixture was cooled to room temperature and the toluene layer was separated. The toluene layer was then washed with water (2 x 250mL), was dried over anhydrous sodium sulfate, and was concentrated under reduced pressure. The resulting residue was purified by distillation under reduced pressure to obtain (3S,4R)-1-benzyl-3-cyclopropylaminomethyl-4-methylpyrrolidine (62.1g) as a colorless oil.

### Step 3:

(3S,4R)-1-Benzyl-3-cyclopropylaminomethyl-4-methylpyrrolidine (25.0g) was dissolved in ethanol (200mL). To this solution, trifluoroacetic acid (15.7mL) and 10% palladium carbon (12.5g) were added and the mixture was stirred at room temperature for 9 hours under a hydrogen pressure of 3.9×10⁵Pa. The catalyst was collected from the reaction mixture by filtration and was washed with ethanol containing 25% water (300mL). The filtrate and the washing solution were combined and the combined solutions were concentrated under reduced pressure. The resultant pale brown crystals were suspended in tetrahydrofuran (100mL) and collected by filtration. The collected crystals were washed with tetrahydrofuran and dried under reduced pressure to give 34.1g of (3R,4R)-3-cyclopropylaminomethyl-4-methylpyrrolidine·trifluoroacetate as white crystals.

### Example 1

### Synthesis of (3R)-10-[(3S)-3-cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid

Process (A): [(3R)-9,10-Difluoro-3-fluoromethyl-2,3-dihydro-7-oxo-7H-pyrido[1,2,3-d,e][1,4]benzoxazine-6-carboxylato-O⁶,O⁷]difluoroboron, (51.0g), 3(R)-cyclopropylaminomethylpyrrolidine (24.7g), triethylamine (24.6mL) and dimethylsulfoxide (500mL) were mixed with one another and the mixture was stirred at 70°C for 1 hour. Subsequently, the reaction mixture was concentrated under reduced pressure and the resulting residue was purified on a silica gel column (eluant = dichloromethane: methanol = 10:1). The eluates were combined and the combined solutions were concentrated under reduced pressure. To the resulting residue, 80% ethanol (2500mL) and triethylamine (25.0mL) were added and the mixture was heat-refluxed for 2 hours while being stirred. Subsequently, the reaction mixture was left on an ice bath for 2 hours and the resulting crystals were collected by filtration. The collected crystals were washed with ethanol, suspended in purified water (300ml), and then collected by filtration. The collected crystals were dried under reduced pressure and purified on a silica gel column (eluant = dichloromethane: methanol = 10:1). The eluates were combined and the combined solutions were concentrated under reduced pressure. The resulting residue was dissolved in ethanol (2000mL) by heating and the solution was allowed to stand for 14 hours at room temperature. The resultant crystals were collected by filtration and the collected crystals were washed with ethanol and dried under reduced pressure to give 27.7g of (3R)-10-[(3S)-3-cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid as a yellow powder.

Process B: To a dichloromethane solution (273mL) of bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (22.6g), (3R)-3-cyclopropylaminomethylpyrrolidine (8.41g) and triethylamine (7.59g) were added and the mixture was allowed to stand at room temperature for 13 hours. Subsequently, the reaction mixture was sequentially washed with water (200mL) and a saturated aqueous solution of,sodium chloride (50ml) was dried over anhydrous sodium sulfate, and was then concentrated under reduced pressure. The resulting residue was purified on a silica gel column (dichloromethane: methanol = 15:1) to obtain a yellow amorphous product. To this product, a 5% aqueous solution of acetic acid (100mL) was added and the mixture was stirred at 80°C for 3 hours.

Subsequently, the reaction mixture was washed with ethyl acetate (100mL). While the mixture was cooled on an ice bath, a 1mol/L aqueous solution of sodium hydroxide was added to adjust the pH to 7.01 and the mixture was further stirred for 0.5 hours. The resultant crystals were collected by filtration, washed with purified water (2 x 50mL), and then dissolved in ethanol (1200mL) by heating. The solution was allowed to stand at room temperature for 12 hours. Subsequently, the resulting crystals were collected by filtration, followed by washing with ethanol and drying under reduced pressure, to give 11.2g of (3R)-10-[(3S)-3-cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid as a yellow crystal.
MS (EI) m/z: 419 (M⁺).
Elementary analysis (%): Calcd. for C₂₁H₂₃F₂N₃O₄: C 60.14, H 5.53, N 10.02; found: C 60.01, H 5.47, N 9.94.

### Example 2

### Synthesis of (3R)-10-[(3R)-3-cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷] boron (982mg) was reacted with (3S)-3-cyclopropylaminomethylpyrrolidine (335mg) to give 587mg of. (3R)-10-[(3R)-3-cyclopropylaminomethyl-1-pyrrolidinyl]-9-fluoro-3-fluoromethyl-2,3-dihydro-7-oxo-7H-pyrido[1,2,3-d,e] [1,4]benzoxazine-6-carboxylic acid as yellow crystals.
MS (FAB⁺) m/z: 420 (MH⁺).
Elementary analysis (%): Calcd. for C₂₁H₂₃F₂N₃O₄· 0.25H₂O: C 59.50, H 5.59, N 9.91; found: C 59.68, H 5.47, N 9.97.

### Example 3

### Synthesis of (3R)-10-[3-cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (513mg) was reacted with 3-cyclopropylaminomethylpyrrolidine (185mg)to give 231mg of (3R)-10-(3-cyclopropylaminomethyl-1-pyrrolidinyl)-9-fluoro-3-fluoromethyl-2,3-dihydro-7-oxo-7H-pyrido[1,2,3-d,e][1,4]benzoxazine-6-carboxylic acid as yellow crystals.
MS (FAB⁺) m/z: 420 (MH⁺).
Elementary analysis (%): Calcd. for C₂₁H₂₃F₂N₃O₄·0.25H₂O: C 59.50, H 5.59, N 9.91: found: C 59.41, H 5.41, N 9.89.

### Example 4 (Reference Example)

### Synthesis of (3S)-10-[(3S)-3-cyclopropylaminomethyl-1-pyrrolidinyl]-9-fluoro-2,3-dihydro-3-methoxymethyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methoxymethyl-7-oxo-7H-pyrido[1,2,3-d,e][1,4]benzoxazine-6-carboxylato-O⁶,O⁷]boron (790mg) was reacted with (3R)-3-cyclopropylaminomethylpyrrolidine (303mg) to give 602mg of (3S)-10-[(3S)-3-cyclopropylaminomethyl-1-pyrrolidinyl]-9-fluoro-2,3-dihydro-3-methoxymethyl-7-oxo-7H-pyrido[1,2,3-d,e][1,4]benzoxazine-6-carboxylic acid as yellow crystals.
MS (FAB⁺) m/z: 432 (MH⁺).
Elementary analysis (%): Calcd. for C₂₂H₂₆FN₃O₅: C 61.24, H 6.07, N 9.74; found: C 61.01, H 6.04, N 9.73.

### Example 5 (Reference Example)

### Synthesis of (3S)-3-acetoxymethyl-10-[(3S)-3-cyclopropylaminomethyl-1-pyrrolidinyl]-9-fluoro-2,3-dihydro-7-oxo-7H-pyrido[1,2,3-d,e][1,4]benzoxazine-6-carboxylic acid

In a similar manner to Process (B) in Example 1, bis(acetato-O)[(3S)-3-acetoxymethyl-9,10-difluoro-2,3-dihydro-7-oxo-7H-pyrido[1,2,3-d,e][1,4]benzoxazine-6-carboxylato-O⁶,O⁷]boron (934mg) was reacted with (3R)-3-cyclopropylaminomethylpyrrolidine (337mg) to give 612mg of (3S)-3-acetoxymethyl-10-[(3S)-3-cyclopropylaminomethyl-1-pyrrolidinyl]-9-fluoro-2,3-dihydro-7-oxo-7H-pyrido[1,2,3-d,e][1,4]benzoxazine-6-carboxylic acid as yellow crystals. MS (FAB⁺) m/z: 460 (MH⁺).
Elementary analysis (%): Calcd. for C₂₃H₂₅FN₃O₆·H₂O: C 57.85, H 5.91, N 8.80; found: C 57.94, H 5.83, N 8.89.

### Example 6 (Reference Example)

### Synthesis of (3S)-10-[(3S)-3-cyclopropylaminomethyl-1-pyrrolidinyl]-9-fluoro-2,3-dihydro-3-hydroxymethyl-7-oxo-7H-pyrido[1,2,3-d,e][1,4]benzoxazine-6-carboxylic acid

A 1mol/L aqueous solution of sodium hydroxide (8.0mL) containing (3S)-3-acetoxymethyl-10-[(3S)-3-cyclopropylaminomethyl-1-pyrrolidinyl]-9-fluoro-2,3-dihydro-7-oxo-7H-pyrido[1,2,3-d,e][1,4]benzoxazine-6-carboxylic acid (368mg) was stirred at 50°C for 2 hours. While this mixture was cooled on an ice bath, 1mol/L hydrochloric acid was added to adjust the pH to 7.05 and the mixture was further stirred for 0.5 hours. The resultant crystals were collected by filtration, washed with purified water, and then dissolved in ethanol (50mL) by heating. The solution was allowed to stand at room temperature for 2 hours. Subsequently, the resulting crystals were collected by filtration, washed ethanol, and then dried under reduced pressure to give 251mg of (3S)-10-[(3S)-3-cyclopropylaminomethyl-1-pyrrolidinyl]-9-fluoro-2,3-dihydro-3-hydroxymethyl-7-oxo-7H-pyrido[1,2,3-d,e][1,4]benzoxazine-6-carboxylic acid as yellow crystals.
MS (FAB⁺) m/z: 418 (MH⁺).
Elementary analysis (%): Calcd. for C₂₁H₂₄FN₃O₅·0.5H₂O: C 59.15, H 5.91, N 9.85; found: C 59.16, H 5.92, N 9.88.

### Example 7 (Reference Example)

### Synthesis of 10-[(3S)-3-cyclopropylaminomethyl-1-pyrrolidinyl]-9-fluoro-2,3-dihydro-3-methylene-7-oxo-7H-pyrido[1,2,3-d,e][1,4]benzoxazine-6-carboxylic acid

(3R)-10-[(3S)-3-Cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid (252mg) was suspended in ethanol (1mL). To this suspension, a 1mol/L aqueous solution of sodium hydroxide (6mL) was added and the mixture was stirred at room temperature for 2 hours. Subsequently, the reaction mixture was concentrated under reduced pressure and the resulting residue was dissolved in purified water (10ml).

While this solution was cooled on an ice bath, 1mol/L hydrochloric acid was added to adjust the pH to 7.03 and the mixture was further stirred for 0.5 hours. The resultant crystals were collected by filtration to obtain 214mg of 10-[(3S)-cyclopropylmethylamino-1-pyrrolidinyl]-9-fluoro-2,3-dihydro-3-methylene-7-oxo-7H-pyrido[1,2,3-d,e][1,4]benzoxazine-6-carboxylic acid as a yellow powder.
MS (FAB⁺) m/z: 400 (MH⁺).
Elementary analysis (%): Calcd. for C₂₂H₂₃FN₃O₄·1.75H₂O: C 58.53, H 5.96, N 9.75; found: C 58.62, H 5.79, N 9.76.

### Example 8

### Synthesis of (3R)-10-[(3S)-cyclopropylaminomethyl-1-pyrrolidinyl]-3-fluoromethyl-2,3-dihydro-7-oxo-7H-pyrido[1,2,3-d,e][1,4]benzoxazine-6-carboxylic acid

In a similar manner to Process (B) in Example 1, bis(acetato-O)[(3R)-10-fluoro-3-fluoromethyl-2,3-dihydro-7-oxo-7H-pyrido[1,2,3-d,e][1,4]benzoxazine-6-carboxylato-O⁶,O⁷]boron (500mg) was reacted with (3R)-3-cyclopropylaminomethylpyrrolidine (240mg) to give 335mg of (3R)-10-[(3S)-3-cyclopropylaminomethyl-1-pyrrolidinyl]-3-fluoromethyl-2,3-dihydro-7-oxo-7H-pyrido[1,2,3-d,e][1,4]benzoxazine-6-carboxylic acid as a yellow needle-shaped product.
MS (FAB⁺) m/z: 402 (MH⁺).
Elementary analysis (%): Calcd. for C₂₁H₂₄FN₃O₄: C 62.83, H 6.. 03, X N 10.47; found: C 62.56, H 5.94, N 10.40.

### Example 9 (Reference Example)

### Synthesis of (3S)-10-[(3S)-cyclopropylaminomethyl-1-pyrrolidinyl]-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-10-fluoro-2,3-dihydro-3-methyl-7-oxo-7H-pyrido[1,2,3-d,e][1,4]benzoxazine-6-carboxylato-O⁶,O⁷]boron (1000mg) was reacted with (3R)-3-cyclopropylaminomethylpyrrolidine (431mg) to.give 335mg of (3S)-10-[(3S)-3-cyclopropylaminomethyl-1-pyrrolidinyl]-2,3-dihydro-3-methyl-7-oxo-7H-pyrido[1,2,3-d,e][1,4]benzoxazine-6-carboxylic acid as a yellow needle-shaped product.
MS (EI⁺) m/z: 383 (M⁺).
Elementary analysis (%): Calcd. for C₂₁H₂₅N₃O4: C 65.78, H 6.57, N 10.96; found: C; 65.58, H 6.61, N 10.91.

### Example 10 (Reference Example)

### Synthesis of (3S)-10-(trans-3-cyclopropylaminomethyl-4-methyl-1-pyrrolidinyl)-9-fluoro-2,3-dihydro-3-methyl-7-oxo-7H-pyrido[1,2,3-d,e] [1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e][1,4]benzoxazine-6-carboxylato-O⁶,O⁷]boron (300mg) was reacted with trans-3-cyclopropylaminomethyl-4-methylpyrrolidine (136mg) to give 166mg of (35)-10-(trans-3-cyclopropylaminomethyl-4-methyl-1-pyrrolidinyl)-9-fluoro-2,3-dihydro-3-methyl-7-oxo-7H-pyrido[1,2,3-d,e][1,4]benzoxazine-6-carboxylic acid as yellow crystals.
MS (EI⁺) m/z: 415 (M⁺).
Elementary analysis (%): Calcd. for C₂₂H₂₆FN₃O₄·0.5H₂O: C 62.25, H 6.41, N 9.90; found: C 62.30, H 6.17, N 10.06.

### Example 11 (Reference Example)

### Synthesis of (3S)-10-[(3S,4R)-3-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido [1,2,3-d,e][1,4]benzoxazine-6-carboxylato-O⁶, O⁷]boron (500mg) was reacted with (3R,4R)-3-cyclopropylamino-4-methylpyrrolidine (226mg) to give 362mg of (3S)-10-[(3S,4R)-3-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid as yellow crystals.
MS (EI⁺) m/z : 415 (M⁺)
Elementary analysis (%): Calcd. for C₂₂H₂₆FN₃O₄: C 63.60, H 6.31, N 10.11; found: C 63.41, H 6.30, N 10.17.

### Example 12 (Reference Example)

### Synthesis of (3S)-10-[(3R,4S)-3-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e][1,4]benzoxazine-6-carboxylato-O⁶,O⁷]boron (500mg) was reacted with (3S,4S)-3-cyclopropylamino-4-methylpyrrolidine (226mg) to give 276mg of (3S)-10-[(3R,4S)-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid as pale yellow crystals.
MS (EI⁺) m/z: 415 (M⁺).
Elementary analysis (%): Calcd. for C₂₂H₂₆FN₃O₄·0.5 H₂O: C 62.25, H 6.41, N 9.90; found: C 62.23, H 6.06, N 9.92.

### Example 13 (Reference Example)

### Synthesis of (3S)-10-(cis-3-cyclopropylaminomethyl-4-methyl-1-pyrrolidinyl)-9-fluoro-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e] [1,4]benzoxazine-6-carboxylato-O⁶,O⁷] boron (220mg) was reacted with cis-3-cyclopropylaminomethyl-4-methylpyrrolidine (100mg) to give 109mg of (3S)-10-(cis-3-cyclopropylaminomethyl-4-methyl-1-pyrrolidinyl)-9-fluoro-2,3-dihydro-3-methyl-7-oxo-7H-pyrido[1,2,3-d,e][1,4]benzoxazine-6-carboxylic acid as yellow crystals.
MS (EI⁺) m/z: 415 (M⁺).
Elementary analysis (%): Calcd. for C₂₂H₂₆FN₃O₄·H₂O: C 60.96, H 6.51, N 9.69; found: C 61.27, H 6.69, N 9.52.

### Example 14 (Reference Example)

### Synthesis of (3S)-10-[(3S,4S)-3-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e] [1,4] benzoxazine-6-carboxylato-O⁶,O⁷] boron (1000mg) was reacted with (3R,4S)-cyclopropylamino-4-methylpyrrolidine (452mg) to give 474mg of (3S)-10-[(3S,4S)-3-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid as yellow crystals.
MS (EI⁺) m/z: 415 (M⁺).
Elementary analysis (%): Calcd. for C₂₂H₂₆FN₃O₄·0.25H₂O: C 62.92, H 6.36, N 10.01; found: C 62.69, H 6.52, N 9.98.

### Example 15 (Reference Example)

### Synthesis of (3S)-10-[(3R,4R)-3-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e][1,4]benzoxazine-6-carboxylato-O⁶,O⁷]boron (250mg) was reacted with (3S,4R)-3-cyclopropylamino-4-methylpyrrolidine (113mg) to give 33mg of (3S)-10-[(3R,4R)-cyclopropylaminomethyl-4-methyl-1-pyrrolidinyl]-9-fluoro-2,3-dihydro-3-methyl-7-oxo-7H-pyrido[1,2,3-d,e][1,4]benzoxazine-6-carboxylic acid as pale yellow crystals.
MS (EI⁺) m/z: 415 (M⁺).
Elementary analysis (%): Calcd. for C₂₂H₂₆FN₃O₄·0.5H₂O: C 62.25, H 6.41, N 9.90; found: C 61.98, H 6.57, N 9.91..

### Example 16

### Synthesis of (3R)-10-(trans-3-cyclopropylaminomethyl-4-methyl-1-pyrrolidinyl)-9-fluoro-3-fluoromethyl-2,3-dihydro-7-oxo-7H-pyrido[1,2,3-d,e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (300mg) was reacted with trans-3-cyclopropylaminomethyl-4-methylpyrrolidine (130mg) to give 110mg of (3R)-10-(trans-3-cyclopropylaminomethyl-4-methyl-1-pyrrolidinyl)-9-fluoro-3-fluoromethyl-2,3-dihydro-7-oxo-7H-pyrido[1,2,3-d,e][1,4]benzoxazine-6-carboxylic acid.
MS (EI⁺) m/z: 433 (M⁺).
Elementary analysis (%): Calcd. for C₂₂H₂₅F₂N₃O₄: C 60.96, H 5.81, N 9.69; found: C 60.81, H 5.85, N 9.66.

### Example 17

### Synthesis of (3R)-10-[(3S,4R)-3-cyclopropylaminomethyl-4-methyl-1-pyrrolidinyl]-9-fluoro-3-fluoromethyl-2,3-dihydro-7-oxo-7H-pyrido[1,2,3-d,e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (1000mg) was reacted with (3R,4R)-cyclopropylamino-4-methylpyrrolidine (397mg) to give 620mg of (3R)-10-[(3S,4R)-3-cyclopropylaminornethyl-4-methyl-1-pyrrolidinyl]-9-fluoro-3-fluoromethyl-2,3-dihydro-7-oxo-7H-pyrido[1,2,3-d,e][1,4]benzoxazine-6-carboxylic acid as yellow crystals. MS (EI⁺) m/z: 433 (M⁺).
Elementary analysis (%): Calcd. for C₂₂H₂₅F₂N₃O₄: C 60.96, H 5.81, N 9.69; found: C 60.81, H 5.86, N 9.63.

### Example 18

### Synthesis of (3R)-10-[(3S,4S)-3-cyclopropylaminomethyl-4-methy-1-pyrrolidinyl]-9-fluoro-3-fluoromethyl-2,3-dihydro-7-oxo-7H-pyrido[1,2,3-d,e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (500mg) was reacted with (3R,4R)-cyclopropylamino-4-methylpyrrolidine (199mg) to give 422mg of (3R)-10-[(3S,4S)-3-cyclopropylaminomethyl-4-methyl-1-pyrrolidinyl]-9-fluoro-3-fluoromethyl-2,3-dihydro-7-oxo-7H-pyrido[1,2,3-d,e][1,4]benzoxazine-6-carboxylic acid as yellow crystals.
MS (EI⁺) m/z: 433 (M⁺).
Elementary analysis (%): Calcd. for C₂₂H₂₅F₂N₃O₄: C 60.96, H 5.81, N 9.69; found: C 60.79, H 5.91, N 9.77.

### Example 19 (Reference Example)

### Synthesis of (3S)-10-(trans-3-cyclopropylaminomethyl-4-trifluoromethyl-1-pyrrolidinyl)-9-fluoro-2,3-dihydro-3-methyl-7-oxo-7H-pyrido[1,2,3-d,e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e][1,4]benzoxazine-6-carboxylato-O⁶,O⁷]boron (300mg) was reacted with trans-3-cyclopropylaminomethyl-4-trifluoromethylpyrrolidine (198mg) to give 87mg of (3S)-10-(trans-3-cyclopropylaminomethyl-4-trifluoromethyl-1-pyrrolidinyl)-9-fluoro-2,3-dihydro-3-methyl-7-oxo-7H-pyrido[1,2,3-d,e][1,4]benzoxazine-6-carboxylic acid.
MS (FAB⁺) m/z: 470 (MH⁺).
Elementary analysis (%) : Calcd. for C₂₂H₂₃F₄N₃O₄: C 56.29, H 4.94, N 8.95; found: C 55.97, H 4.84, N 9.00.

### Example 20

### Synthesis of (3R)-10-[(3S,4S)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (500mg) was reacted with (3R,4S)-3-cyclopropylaminomethyl-4-fluoropyrrolidine (204mg) to give 387mg of (3R)-10-[(3S,4S)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid as pale yellow crystals.
MS (FAB⁺): m/z = 438 (MH⁺).
Elementary analysis (%): Calcd. for C₂₁H₂₂F₃N₃O₄: C 57.66, H 5.07, N 9.61; found: C 57.47, H 5.07, N 9.57.

### Example 21 (Reference Example)

### Synthesis of (3S)-10-[(3S,4S)-3-cyclopropylamino-4-fluoro-1-pyrrolidinyl]-9-fluoro-2,3-dihydro-3-methyl-7-oxo-7H-pyrido[1,2,3-d.e][1,4]benzoxazine-6-carboxylic acid

In a similar manner to Process (B) in Example 1, bis(acetato-O)[(3S)-9,10-difluoro-2,3-dihydro-3-methyl-7-oxo-7H-pyrido[1,2,3-d,e][1,4]benzoxazine-6-carboxylato-O⁶,O⁷]boron (64.6mg) was reacted with (3R,4S)-3-cyclopropylaminomethyl-4-fluoropyrrolidine (25.0mg) to give 18.5mg of (3R)-10-[(3S,4S)-3-cyclopropylamino-4-fluoro-1-pyrrolidinyl]-9-fluoro-2,3-dihydro-3-methyl-7-oxo-7H-pyrido[1,2,3-d.e][1,4]benzoxazine-6-carboxylic acid as a yellow powder.
MS (FAB⁺) m/z = 20 (MH⁺).
HRMS (FAB⁺): Calcd. for C₂₁H₂₄F₂N₃O₄: 420.1735; found: 420.1747.

### Example 22

### Synthesis of (3R)-10-[(3S,4S)-3-[(N-tert-butoxycarbonyl-N-cyclopropyl)amino]methyl-4-fluoromethyl-1-pyrrolidine]-9-fluoro-3-fluoromethyl-2,3-dihydro-7-oxo-7H-pyrido[1,2,3-d.e][1,4]benzoxazine-6-carboxylic acid

Bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷] boron (912mg), (3R,4S)-3-[(N-tert-butoxycarbonyl-N-cyclopropyl)amino]methyl-4-fluoromethylpyrrolidine (640mg), triethylamine (0.33mL) and acetonitrile (17mL) were mixed with one another and the mixture was stirred at 60°C for 90min. Subsequently, the reaction mixture was concentrated under reduced pressure and the resulting residue was purified on a silica gel column (eluant = ethyl acetate: methanol = 20:1). To the eluate, a 5% aqueous solution of acetic acid (17mL) and ethanol (10mL) were added and the mixture was stirred at 80°C for 2 hours. The mixture was allowed to cool and the resulting crystals were collected by filtration, was washed with a mixture of water and ethanol, and then dried under reduced pressure to give 915mg of (3R)-10-[(3S,4S)-3-[(N-tert-butoxycarbonyl-N-cyclopropyl)amino]methyl-4-fluoromethyl-1-pyrrolidine]-9-fluoro-3-fluoromethyl-2,3-dihydro-7-oxo-7H-pyrido [1,2,3-d.e][1,4]benzoxazine-6-carboxylic acid as a yellow powder.
MS (EI) m/z = 551 (M⁺).
Elementary analysis (%): Calcd. for C₂₇H₃₂F₃N₃O6·0.5H₂O: C 57.85, H 5.93, N 7.50; found: C 57.90, H 5.80, N 7.49.

### Example 23

### Synthesis of (3R)-10-[(3S,4S)-3-cyclopropyl]amino]methyl-4-fluoromethyl-1-pyrrolidinyl]-9-fluoro-3-fluoromethyl-2,3-dihydro-7-oxo-7H-pyrido[1,2,3-d.e][1,4]benzoxazine-6-carboxylic acid hydrochloride

(3R)-10-[(3S,4S)-3-[(N-tert-Butoxycarbonyl-N-cyclopropyl)amino]methyl-4-fluoromethyl-1-pyrrolidine]-9-fluoro-3-fluoromethyl-2,3-dihydro-7-oxo-7H-pyrido[1,2,3-d.e][1,4]benzoxazine-6-carboxylic acid (860mg) was dissolved in ethanol (9mL) saturated with hydrogen chloride. The mixture was stirred at room temperature for 1 hour and was subsequently concentrated under reduced pressure. To the resulting residue, ethanol (50mL) was added and the mixture was concentrated under reduced pressure. After repeating the ethanol addition and concentration once, ethanol (50mL) was added to the resultant residue, and the mixture was heated to 70°C and was then allowed to stand at room temperature for 1 hour. The resulting crystals were collected by filtration, followed by washing with ethanol and drying under reduced pressure, to give 762mg of (3R)-10-[(3S,4S)-3-cyclopropyl]amino]methyl-4-fluoromethyl-1-pyrrolidinyl]-9-fluoro-3-fluoromethyl-2,3-dihydro-7-oxo-7H-pyrido[1,2,3-d.e][1,4]benzoxazine-6-carboxylic acid hydrochloride as yellow crystals.
MS (FAB⁺): m/z = 452 (MH⁺).
Elementary analysis (%): Calcd. for C₂₂H₂₄F₃N₃O₄·HCl·H₂O·0.5C₂H₅OH: C 52.23, H 5.72, N 7.94; found: C 52.17, H 5.38, N 8.20.

### Example 24

### Synthesis of (3R)-10-[(3S,4R)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid

In a similar manner to Process (B) in Example 1, bis(acetato-O)[(3R)-9,10-difluoro-3-fluoromethyl-2,3-dihydro-7-oxo-7H-pyrido[1,2,3-d,e][1,4]benzoxazine-6-carboxylato-O⁶,O⁷]boron (100mg) was reacted with (3R,4R)-3-cyclopropylaminomethyl-4-fluoropyrrolidine (40.6mg) to give 71.0mg of (3R)-10-[(3S,4R)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid as pale yellow crystals.
MS (FAB⁺): m/z = 438 (MH⁺).
Elementary analysis (%): Calcd. for C₂₁H₂₂F₃N₃O₄: C 57.66, H 5.07, N 9.61; found: C 57.50, H 5.18, N 9.22.

### Example 25

### Synthesis of (3R)-10-[(3S,4S)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid methanesulfonate

(3R)-10-[(3S,4S)-3-Cyclopropylaminomethyl-4-fluoro-1-pyrrolidinyl]-9-fluoro-3-fluoromethyl-2,3-dihydro-7-oxo-7H-pyrido[1,2,3-d.e][1,41benzoxazine-6-carboxylic acid (50.0mg) was suspended in ethanol (2mL). To this suspension, methanesulfonic acid (15.0µL) was added and the mixture was stirred at room temperature for 1 hour. The resulting crystals were collected by filtration, washed with ethanol, and then dried under reduced pressure to give 50.4mg of (3R)-10-[(3S,4S)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid methanesulfonate as pale yellow crystals.
MS (FAB⁺): m/z = 438 (MH⁺).
Elementary analysis (%): Calcd. for C₂₁H₂₂F₃N₃O₄·CH₃SO₃H·0.25H₂O: C 49.11, H 4.96, N 7.81; found: C 49.18, H 4.86, N 7.42.

### Example 26

### Synthesis of (3R)-10-[(3S,4S)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid hydrochloride

(3R)-10-[(3S,4S)-3-Cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid (50.0mg) was suspended in ethanol (2mL). To this suspension, ethanol (60.0µL) saturated with hydrogen chloride was added and the mixture was stirred at room temperature for 1 hour. The resulting crystals were collected by filtration, washed with ethanol, and then dried under reduced pressure to give 52.9mg of (3R)-10-[(3S,4S)-3-cyclopropylaminomethyl-4-fluoro-1-pyrrolidinyl]-9-fluoro-3-fluoromethyl-2,3-dihydro-7-oxo-7H-pyrido[1,2,3-d.e][1,4]benzoxazine-6-carboxylic acid hydrochloride as pale yellow crystals.
MS (FAB⁺): m/z = 438 (MH⁺).
Elementary analysis (%): Calcd. for C₂₁H₂₂F₃N₃O₄·HCl·0.25H₂O: C 52.73, H 4.95, N 8.78; found: C 52.68, H 5.04, N 8.28. (Antibacterial activity)

### Test Example 1: in vitro antibacterial activity

The *in vitro* antibacterial activity (as measured by the minimum inhibitory concentration (MIC)) was determined for each of the compounds of the present invention by the agar dilution method according to NCCLS (National Committee for Clinical Laboratory Standard (1997), Methods for Dilution Antibacterial Susceptibility Tests for Bacteria that grow Aerobically-Forth Edition: Approved Standard m7-A4. NCCLS, Villanova, Pa.), which involved the use of Muller-Hinton agar medium. For pneumococci and enterococci, MIC was determined by using Muller-Hinton agar medium containing 5% defibrinated equine blood. The results are shown in Table 1 below

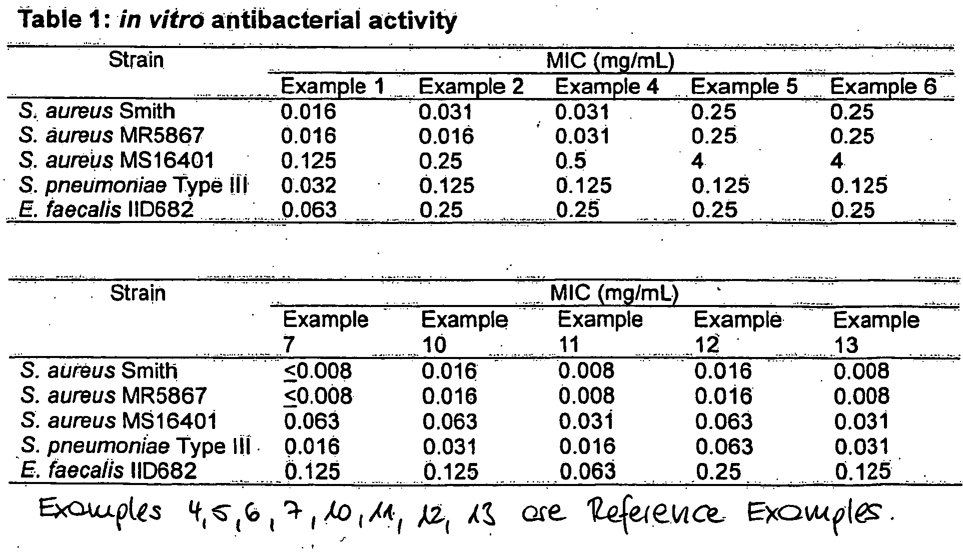

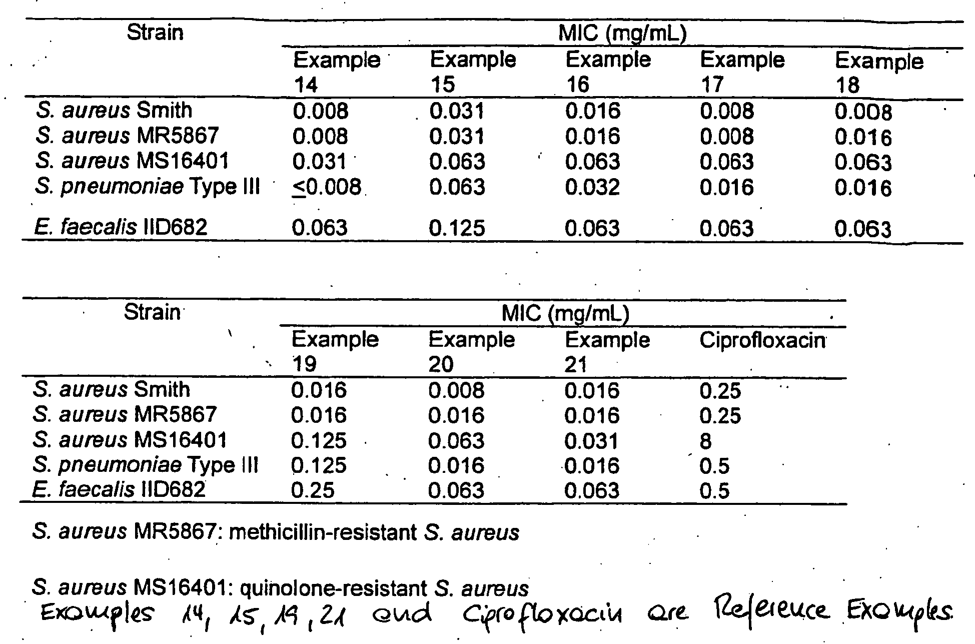

The novel 10-(3-cyclopropylaminomethyl-1-pyrrolidinyl)pyridobenzoxazine carboxylic acid derivatives, salts and hydrates thereof, which are compounds of the present invention, are not only safe and exhibit strong antibacterial activities, but they are also effective against drug-resistant bacteria that are less susceptible to conventional antibacterial agents.

## Claims

1. A pyridobenzoxazine carboxylic acid derivative as represented by the following general formula (I), or a salt or a hydrate thereof: 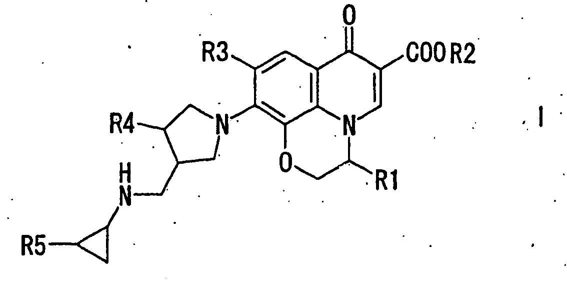 wherein R1 is a fluoromethyl group; R2 is a hydrogen atom, a lower alkyl group having 1 to 3 carbon atoms, or a pharmaceutically acceptable cation and an ester of a prodrug selected from a pivaloyloxymethyl group, an acetoxymethyl group, a phthalidinyl group, an indanyl group, a methoxymethyl group, and a 5-methyl-2-oxo-1,3- dioxolene-4-yl group; R3 is a hydrogen atom or a halogen atom; R4 is a hydrogen atom, a lower alkyl group having 1 to 3 carbon atoms, a fluoromethyl group, a trifluoromethyl group or a fluorine atom; and R5 is a hydrogen atom or a fluorine atom.

2. The compound according to claim 1, a salt or a hydrate thereof, wherein in the general formula (I), R3 is a fluorine atom.

3. The compound according to claim 1, a salt or a hydrate thereof, wherein in the general formula (I), R3 is a fluorine atom, and R4 is a hydrogen atom, a methyl group, a fluoromethyl group or a fluorine atom.

4. The compound according to claim 1, a salt or a hydrate thereof, wherein in the general formula (I), R3 is a fluorine atom, and R4 is a hydrogen atom, a methyl group, a fluoromethyl group or a fluorine atom.

5. The compound according to any one of claims 1 to 4, a salt or a hydrate thereof, wherein the compound of the general formula (I) has a single stereochemistry.

6. The compound according to claim 1, a salt or a hydrate thereof, wherein the compound of the general formula (I) is (3R)-10-[(3S,4R)-3-cyclopropylaminomethyl-4-methyl-1-pyrrolidinyl]-9-fluoro-3-fluoromethyl-2,3-dihydro-7-oxo-7H-pyrido[1,2,3-d,e][1,4]benzoxazine-6-carboxylic acid, a salt or a hydrate thereof.

7. The compound according to claim 1, a salt or a hydrate thereof, wherein the compound of the general formula (I) is (3R)-10-[(3S,4S)-3-cyclopropylaminomethyl-4-methyl-1-pyrrolidinyl]-9-fluoro-3-fluoromethyl-2,3-dihydro-7-oxo-7H-pyrido[1,2,3-d,e][1,4]benzoxazine-6-carboxylic acid, a salt or a hydrate thereof.

8. The compound according to claim 1, a salt or a hydrate thereof, wherein the compound of the general formula (I) is (3R)-10-[(3S)-3-cyclopropylaminomethyl-1-pyrrolidinyl]-9-fluoro-3-fluoromethyl-2,3-dihydro-7-oxo-7H-pyrido[1,2,3-d,e][1,4]benzoxazine-6-carboxylic acid, a salt or a hydrate thereof.

9. The compound according to claim 1, a salt or a hydrate thereof, wherein the compound of the general formula (I) is (3R)-10-[(3R)-3-cyclopropylaminomethyl-1-pyrrolidinyl]-9-fluoro-3-fluoromethyl-2,3-dihydro-7-oxo-7H-pyrido[1,2,3-d,e][1,4] benzoxazine-6-carboxylic acid, a salt or a hydrate thereof.

10. The compound according to claim 1, a salt or a hydrate thereof, wherein the compound of the general formula (I) is (3R)-10-[(3S,4R)-cyclopropylaminomethyl-4-fluoro-1-pyrrolidinyl]-9-fluoro-3-fluoromethyl-2,3-dihydro-7-oxo-7H-pyrido[1,2,3-d,e][1,4]benzoxazine-6-carboxylic acid, a salt or a hydrate thereof.

11. The compound according to claim 1, a salt or a hydrate thereof, wherein the compound of the general formula (I) is (3R)-10-[(3S,4S)-cyclo.propylaminomethyl-4-fluoro-1-pyrrolidinyl]-9-fluoro-3-fluoromethyl-2,3-dihydro-7-oxo-7H-pyrido[1,2,3-d,e][1,4]benzoxazine-6-carboxylic acid, a. salt or a hydrate thereof.

12. An antibacterial agent containing as an active ingredient the compound according to any one of claims 1 to 11, a salt or a hydrate thereof.

## Patentansprüche

1. Pyridobenzoxazincarbonsäurederivat, dargestellt durch die folgende allgemeine Formel (I), oder ein Salz oder Hydrat davon: 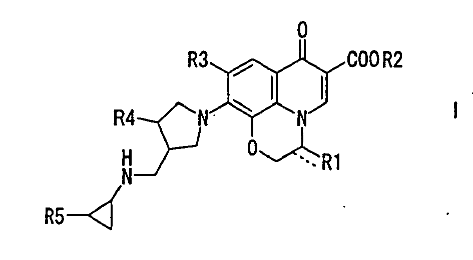 wobei R1 ein Fluormethylrest ist; R2 ein Wasserstoffatom, ein Niederalkylrest mit 1 bis 3 Kohlenstoffatomen oder ein pharmazeutisch verträgliches Kation und ein Ester eines Prodrugs, ausgewählt aus einem Pivaloyloxymethylrest, einem Acetoxymethylrest, einem Phthalidinylrest, einem Indanylrest, einem Methoxymethylrest und einem 5-Methyl-2-oxo-1,3-dioxolen-4-ylrest, ist; R3 ein Wasserstoffatom oder ein Halogenatom ist; R4 ein Wasserstoffatom, ein Niederalkylrest mit 1 bis 3 Kohlenstoffatomen, ein Fluormethylrest, ein Trifluormethylrest oder ein Fluoratom ist; und R5 ein Wasserstoffatom oder ein Fluoratom ist.

2. Verbindung gemäß Anspruch 1, ein Salz oder ein Hydrat davon, wobei in der allgemeinen Formel (I) R3 ein Fluoratom ist.

3. Verbindung gemäß Anspruch 1, ein Salz oder ein Hydrat davon, wobei in der allgemeinen Formel (I) R3 ein Fluoratom ist und R4 ein Wasserstoffatom, eine Methylgruppe, ein Fluormethylrest oder ein Fluoratom ist.

4. Verbindung gemäß Anspruch 1, ein Salz oder ein Hydrat davon, wobei in der allgemeinen Formel (I) R3 ein Fluoratom ist und R4 ein Wasserstoffatom, eine Methylgruppe, ein Fluormethylrest oder ein Fluoratom ist.

5. Verbindung gemäß einem der Ansprüche 1 bis 4, ein Salz oder ein Hydrat davon, wobei die Verbindung der allgemeinen Formel (I) eine einzige Stereochemie aufweist.

6. Verbindung gemäß Anspruch 1, ein Salz oder ein Hydrat davon, wobei die Verbindung der allgemeinen Formel (I) (3R)-10-[(3S,4R)-3-Cyclopropylaminomethyl-4-methyl-1-pyrrolidinyl]-9-fluor-3-fluormethyl-2,3-dihydro-7-oxo-7H-pyrido[1,2,3-d,e] [1,4]benzoxazin-6-carbonsäure, ein Salz oder ein Hydrat davon ist.

7. Verbindung gemäß Anspruch 1, ein Salz oder ein Hydrat davon, wobei die Verbindung der allgemeinen Formel (I) (3R)-10-[(3S,4S)-3-Cyclopropylaminomethyl-4-methyl-1-pyrrolidinyl]-9-fluor-3-fluormethyl-2,3-dihydro-7-oxo-7H-pyrido[1,2,3-d,e][1,4]benzoxazin-6-carbonsäure, ein Salz oder ein Hydrat davon ist.

8. Verbindung gemäß Anspruch 1, ein Salz oder ein Hydrat davon, wobei die Verbindung der allgemeinen Formel (I) (3R)-10-[(3S)-3-Cyclopropylaminomethyl-1-pyrrolidinyl]-9-fluor-3-fluormethyl-2,3-dihydro-7-oxo-7H-pyrido[1,2,3-d,e] [1,4]benzoxazin-6-carbonsäure, ein Salz oder ein Hydrat davon ist.

9. Verbindung gemäß Anspruch 1, ein Salz oder ein Hydrat davon, wobei die Verbindung der allgemeinen Formel (I) (3R)-10-[(3R)-3-Cyclopropylaminomethyl-1-pyrrolidinyl]-9-fluor-3-fluormethyl-2,3-dihydro-7-oxo-7H-pyrido[1,2,3-d,e][1,4]benzoxazin-6-carbonsäure, ein Salz oder ein Hydrat davon ist.

10. Verbindung gemäß Anspruch 1, ein Salz oder ein Hydrat davon, wobei die Verbindung der allgemeinen Formel (I) (3R)-10-[(3S,4R)-Cyclopropylaminomethyl-4-fluor-1-pyrrolidinyl]-9-fluor-3-fluormethyl-2,3-dihydro-7-oxo-7H-pyrido[1,2,3-d,e][1,4]benzoxazin-6-carbonsäure, ein Salz oder ein Hydrat davon ist.

11. Verbindung gemäß Anspruch 1, ein Salz oder ein Hydrat davon, wobei die Verbindung der allgemeinen Formel (I) (3R)-10-[(3S,4S)-Cyclopropylaminomethyl-4-fluor-1-pyrrolidinyl]-9-fluor-3-fluormethyl-2,3-dihydro-7-oxo-7H-pyrido[1,2,3-d,e][1,4]benzoxazin-6-carbonsäure, ein Salz oder ein Hydrat davon ist.

12. Antibakterielles Mittel, welches als Wirkstoff die Verbindung gemäß einem der Ansprüche 1 bis 11, ein Salz oder Hydrat davon enthält.

## Revendications

1. Dérivé d'acide carboxylique pyridobenzoxazine tel que représenté par la formule générale (I) suivante, ou sel ou hydrate de celui-ci : 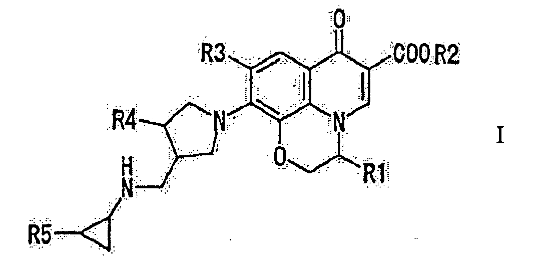 dans laquelle R¹ est un groupe fluorométhyle ; R² est un atome d'hydrogène, un groupe alkyle inférieure ayant de 1 à 3 atomes de carbone, ou un cation acceptable sur le plan pharmaceutique et un ester d'un promédicament choisi parmi un groupe pivaloyloxyméthyle, un groupe acétoxyméthyle, un groupe phtalidinyle, un groupe indanyle, un groupe méthoxyméthyle, et un groupe 5-méthyl-2-oxo-1,3-dioxolène-4-yl ; R³ est un atome d'hydrogène ou un atome d'halogène ; R⁴ est un atome d'hydrogène, un groupe alkyle inférieur ayant de 1 à 3 atomes de carbone, un groupe fluorométhyle, un groupe trifluorométhyle ou un atome de fluor ; et R⁵ est un atome d'hydrogène ou un atome de fluor.

2. Composé selon la revendication 1, sel ou hydrate de celui-ci, dans lequel dans la formule générale (I) R³ est un atome de fluor.

3. Composé selon la revendication 1, sel ou hydrate de celui-ci, dans lequel dans la formule générale (I), R³ est un atome de fluor et R⁴ est un atome d'hydrogène, un groupe méthyle, un groupe fluorométhyle ou un atome de fluor.

4. Composé selon la revendication 1, sel ou hydrate de celui-ci, dans lequel dans la formule générale (I), R³ est un atome de fluor, et R⁴ est un atome d'hydrogène, un groupe méthyle, un groupe fluorométhyle ou un atome de fluor.

5. Composé selon l'une quelconque des revendications 1 à 4, sel ou hydrate de celui-ci, dans lequel le composé de formule générale (I) a une stéréochimie unique.

6. Composé selon la revendication 1, sel ou hydrate de celui-ci, dans lequel le composé de formule générale (I) est l'acide (3R)-10-[(3S,4R)-3-cyclopropylaminométhyl-4-méthyl-1-pyrrolidinyl]-9-fluoro-3-fluorométhyl-2,3-dihydro-7-oxo-7H-pyrido[1,2,3-d,e][1,4]benzoxazine-6-carboxylique, un sel ou un hydrate de celui-ci.

7. Composé selon la revendication 1, sel ou hydrate de celui-ci, dans lequel le composé de formule générale (I) est l'acide (3R)-10-[(3S,4S)-3-cyclopropylaminométhyl-4-méthyl-1-pyrrolidinyl]-9-fluoro-3-fluorométhyl-2,3-dihydro-7-oxo-7H-pyrido[1,2,3-d,e][1,4]benzoxazine-6-carboxylique, un sel ou un hydrate de celui-ci.

8. Composé selon la revendication 1, sel ou hydrate de celui-ci, dans lequel le composé de formule générale (I) est l'acide (3R)-10-[(3S)-3-cyclopropylaminométhyl-1-pyrrolidinyl]-9-fluoro-3-fluorométhyl-2,3-dihydro-7-oxo-7H-pyrido[1,2,3-d,e][1,4]benzoxazine-6-carboxylique, un sel ou un hydrate de celui-ci.

9. Composé selon la revendication 1, sel ou hydrate de celui-ci, dans lequel le composé de formule générale (I) est l'acide (3R)-10-[(3R)-3-cyclopropylaminométhyl-1-pyrrolidinyl]-9-fluoro-3-fluorométhyl-2,3-dihydro-7-oxo-7H-pyrido[1,2,3-d,e][1,4]benzoxazine-6-carboxylique, un sel ou un hydrate de celui-ci.

10. Composé selon la revendication 1, sel ou hydrate de celui-ci, dans lequel le composé de formule générale (I) est l'acide (3R)-10-[(3S,4R)-cyclopropylaminométhyl-4-fluoro-1-pyrrolidinyl]-9-fluoro-3-fluorométhyl-2,3-dihydro-7-oxo-7H-pyrido[1,2,3-d,e][1,4]benzoxazine-6-carboxylique, un sel ou un hydrate de celui-ci.

11. Composé selon la revendication 1, sel ou hydrate de celui-ci, dans lequel le composé de formule générale (I) est l'acide (3R)-10-[(3S,4S)-cyclopropylaminométhyl-4-fluoro-1-pyrrolidinyl]-9-fluoro-3-fluorométhyl-2,3-dihydro-7-oxo-7H-pyrido[1,2,3-d,e][1,4]benzoxazine-6-carboxylique, un sel ou un hydrate de celui-ci.

12. Agent antibactérien contenant, en tant que principe actif, le composé selon l'une quelconque des revendications 1 à 11, un sel ou un hydrate de celui-ci.
